# EUROPEAN PATENT SPECIFICATION

(11) **EP 0 633 887 B1**
(45) Date of publication and mention of the grant of the patent: **19.05.1999**
(21) Application number: 93908587.4
(22) Date of filing: 24.03.1993
(51) Int. Cl.: C07D 493/14, A61K 31/35

(54) **CALANOLIDE ANTIVIRAL COMPOUNDS, COMPOSITIONS AND USES THEREOF**
CALANOLID ANTIVIRALE VERBINDUNGEN, ZUSAMMENSETZUNGEN UND VERWENDUNGEN DAVON
COMPOSES ANTIVIRAUX CALANOLIDES, COMPOSITIONS ISSUES DE CES COMPOSES ET UTILISATIONS

(30) Priority: 31.03.1992 US 861249
(43) Date of publication of application: 18.01.1995
(73) Proprietor: THE UNITED STATES OF AMERICA, as represented by the Secretary of the Department of Health and Human Services, Bethesda, MD 20892-9902 (US)
(72) Inventor: BOYD, Michael, R., Ijamsville, MD 21754 (US); CARDELLINA, John, H., II, Walkersville, MD 21793 (US); GUSTAFSON, Kirk, R., Mt. Airy, MD 21771 (US); McMAHON, James, B., Frederick, MD 21701 (US); FULLER, Richard, W., Tracy's Landing, MD 20779 (US); CRAGG, Gordon, M., Bethesda, MD 20814 (US); KASHMAN, Yoel, 64 372 Tel Aviv (IL)
(74) Representative: Paget, Hugh Charles Edward
(86) International application number: US9302810
(87) International publication number: WO9320082

(56) References cited:
- WO-A-92/06695
- JOURNAL OF THE CHEMICAL SOCIETY, PERKIN TRANSACTIONS 1. no. 13, 1977, LETCHWORTH GB pages 1505 - 1511 S. P. GUNASEKERA ET AL 'Chemical investigation of ceylonese plants. Part 27. Extractives of Calophyllum cuneifolium Thw. and Calophyllum soulattri Burm. f.'
- JOURNAL OF ORGANIC CHEMISTRY. vol. 29, no. 12, 1964, EASTON US pages 3604 - 3609 G. H. STOUT ET AL 'The structure of costatolide'
- CHEMICAL ABSTRACTS, vol. 78, no. 3, 22 January 1973, Columbus, Ohio, US; abstract no. 13744f, KAZUYOSHI KAWAZU ET AL 'Piscicidal constituents of Capophyllum inophyllum' page 207 ;
- PHYTOCHEMISTRY vol. 24, no. 7, 1985, OXFORD GB pages 1553 - 1556 H. R. W. DHARMARATNE ET AL 'Triterpenoids and coumarins from the leaves of Calophyllum cordato-oblongum'
- RESEARCH COMMUNICATIONS IN CHEMICAL PATHOLOGY AND PHARMACOLOGY vol. 9, no. 1, September 1974, WESTBURY, N.Y., US pages 11 - 22 A. K. CHATURVEDI ET AL 'Anticonvulsant and antiinflammatory activity of natural plant coumarins and triterpenoids'
- JOURNAL OF MEDICINAL CHEMISTRY. vol. 35, no. 15, 24 July 1992, WASHINGTON US pages 2735 - 2743 Y. KASHMAN ET AL 'The calanolides, a novel HIV-inhibitory class of coumarin derivatives from the tropical rainforest tree Callophyllum lanigerum'

## Description

### Technical Field of the Invention

This invention relates to antiviral compounds, in particular antiviral compounds isolated from plants of the genus Calophyllum, specifically compounds referred to as calanolides. This invention also relates to methods of isolating calanolides from Calophyllum plants, compositions comprising calanolides, and methods of using the compositions in clinical applications, such as antiviral therapy and the prevention of viral infection.

### Background of the Invention

Acquired immune deficiency syndrome (AIDS) is a very serious disease, reported cases of which have increased dramatically within the past several years. Estimates of reported cases in the very near future also continue to rise dramatically. Consequently, there is a great effort to develop drugs and vaccines to combat AIDS.

The AIDS virus was first identified in 1983. It has been known by several names and acronyms. It is the third known T-lymphocyte virus (HTLV-III), and it has the capacity to replicate within cells of the immune system, causing profound cell destruction. The AIDS virus is a retrovirus, a virus that uses reverse transcriptase during replication. This particular retrovirus is also known as lymphadenopathy-associated virus (LAV), AIDS-related virus (ARV) and, most recently, as human immunodeficiency virus (HIV). Two distinct types of HIV have been described to date, namely HIV-1 and HIV-2. The acronym HIV will be used herein to refer to HIV viruses generically.

Specifically, HIV is known to exert a profound cytopathic effect on the CD4+ helper/inducer T-cells, tnereby severely compromising the immune system. HIV infection also results in neurological deterioration and, ultimately, in the death of the infected individual.

The field of viral chemotherapeutics has developed in response to the need for agents effective against retroviruses, in particular HIV. There are many ways in which an agent can exhibit anti-retroviral activity. For example, HIV requires at least four viral proteins for replication: reverse transcriptase (RT), protease, transactivator protein (TAT), and regulator of virion-protein expression (REV). Accordingly, viral replication could theoretically be inhibited through inhibition of any one or all of the proteins involved in viral replication.

Anti-retroviral agents, such as AZT and ddC, are known to inhibit RT. There also exist anti-viral agents that inhibit TAT.

Nucleoside derivatives, such as AZT, are the only clinically active agents that are currently available for antiviral therapy. Although very useful, the utility of AZT and related compounds is limited by toxicity and insufficient therapeutic indices for fully adequate therapy.

Synthetic peptides also are being developed for potential use as inhibitors of the retroviral protease in the treatment of AIDS. Although these inhibitors are effective in preventing the retroviral protease from functioning, the inhibitors suffer from some distinct disadvantages. First of all, since the active site of the protease is hindered, i.e., has reduced accessibility as compared to the remainder of the protease, the ability of the inhibitors to access and bind in the active site of the protease is impaired. Secondly, the peptide inhibitors that bind to the active site of the protease are generally poorly soluble in water, causing distinct problems in drug delivery.

Therefore, new classes of antiviral agents to be used alone or in combination with AZT and/or other agents are urgently needed for effective antiviral therapy against HIV. New agents, which may be used to prevent HIV infection, are also important.

### Brief Summary Of The Invention

It is an object of the present invention to provide novel antiviral compounds, in particular antiviral compounds isolated from plants of the genus Calophyllum, specifically compounds referred to as calanolides and derivatives thereof.

It is another object of the present invention to provide a method of isolating novel antiviral compounds, specifically calanolides and derivatives thereof, from plants of the genus Calophyllum, specifically Calophyllum lanigerum.

It is still another object of the present invention to provide a composition, in particular a pharmaceutical composition, which inhibits the growth or replication of a virus, such as a retrovirus, in particular a human immunodeficiency virus, specifically HIV-1 or HIV-2.

It is an additional object of the present invention to provide a composition, in particular a pharmaceutical composition, which prevents infection of an animal, in particular a human, with a virus, such as a retrovirus, in particular a human immunodeficiency virus, specifically HIV-1 or HIV-2.

Yet another object of the present invention is to provide a method of treating an animal, in particular a human, infected with a virus, such as a retrovirus, in particular a human immunodeficiency virus, specifically HIV-1 or HIV-2.

A further object of the present invention is to provide a method of treating an animal, in particular a human, to prevent infection with a virus, such as a retrovirus, in particular a human immunodeficiency virus, specifically HIV-1 or HIV-2.

These and other objects of the present invention, as well as additional inventive features, will become apparent from the description herein.

The present invention provides novel antiviral compounds, in particular antiviral compounds isolated from plants of the genus Calophyllum, specifically compounds referred to as calanolides and related derivatives in substantially pure form. The present invention also provides for a method of isolating and purifying calanolides and related derivatives from Calophyllum plants, in particular Calophyllum lanigerum. The isolated compounds may be used in a composition, such as a pharmaceutical composition, which may additionally comprise one or more other antiviral agents. Such a composition has been found to inhibit the growth or replication of a virus, in particular a retrovirus, specifically a human immunodeficiency virus, such as HIV-1 or HIV-2. The composition, therefore, is expected to have utility in the therapeutic treatment of an animal, such as a human, infected with a virus, particularly a retrovirus, specifically a human immunodeficiency virus, such as HIV-1 or HIV-2, and in the prophylactic treatment of an animal, such as a human, to prevent viral infection.

### Brief Description of the Drawings

Figure 1 illustrates the structures of calanolides and related derivatives (compounds **1 - 8**) isolated from Calophyllum lanigerum. Compound **1** is calanolide A, and compound **4** is calanolide B.

Figure 2 illustrates previously known structures (compounds **9 - 13**) reported from other sources.

Figure 3 shows the ¹H-NMR values (△δ = δₛ-δ_{R} in Hertz at 500 MHz) for (R)- and (S)-MTPA esters of calanolide A (**1**) and calanolide B (**4**) used in the determination of the absolute configuration of calanolides A and B.

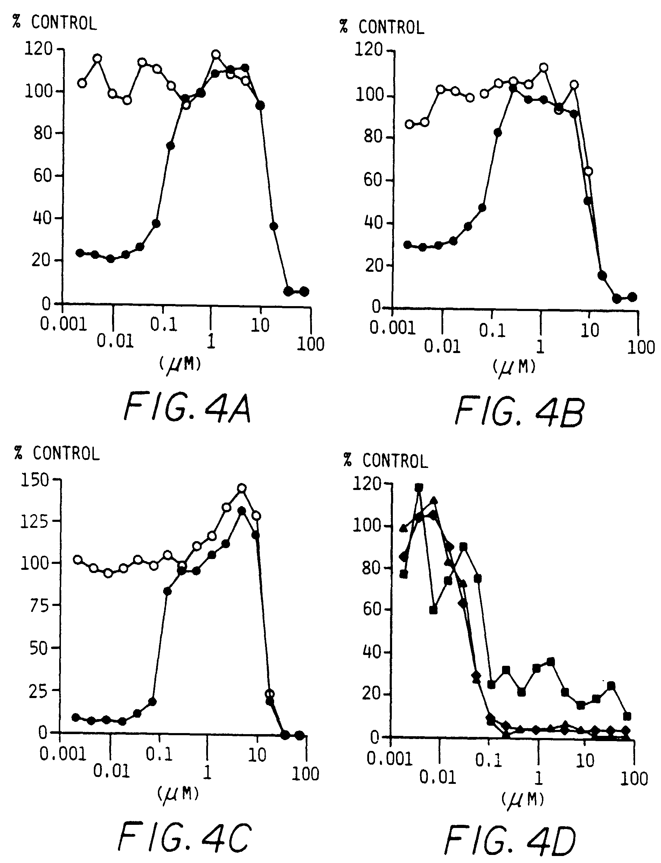

Figure 4, A-D, shows an example of anti-HIV-1 activity of a calanolide. Figures 4A, 4B, and 4C show the effects of a range of concentrations of calanolide A upon uninfected CEM-SS cells (o) and upon CEM-SS cells infected with HIV-1 (^{•}), as determined after 6 days in culture; Fig. 4A depicts the relative numbers of viable CEM-SS cells, as assessed by the BCECF assay; Fig. 4B depicts the relative DNA content of the respective cultures; Fig. 4C depicts the relative numbers of viable CEM-SS cells, as assessed by the XTT assay. Fig. 4D shows the effects of a range of concentrations of calanolide A upon indices of infectious virus or viral replication; these indices include viral reverse transcriptase (▲), viral core protein p24 (◆) and syncytium-forming units (■). In Figs. 4A, 4B, and 4C, the data points are represented as the percent of the uninfected, non-drug treated control values. In Fig. 4D the data points are represented as the percent infected, non-drug treated control values.

Figure 5 more generally illustrates calanolides and derivatives thereof (series 1) and 7,8-dihydrocalanolides and derivatives thereof (series 2), wherein R¹ is C₁-C₆ alkyl or aryl; R² is 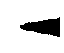 OH, 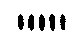 OH, 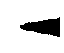 OR³, 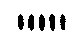 OR³, 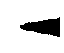 O₂CR³, 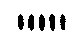 O₂CR³, 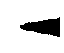 O₃SR³, or 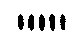 O₃SR³, wherein R³ is C₁-C₆ alkyl or aryl; and R⁴ and R⁵ are the same or different and are each 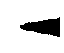 CH₃ or 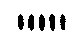 CH₃. ( 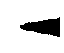 indicates a bond that extends out of the plane of the paper toward the reader, whereas 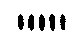 indicates a bond that extends into the plane of the paper away from the reader.)

### Detailed Description of the Invention

The present invention provides novel antiviral compounds (hereinafter referred to as "calanolides"), and derivatives thereof, in substantially pure form and having the structures: 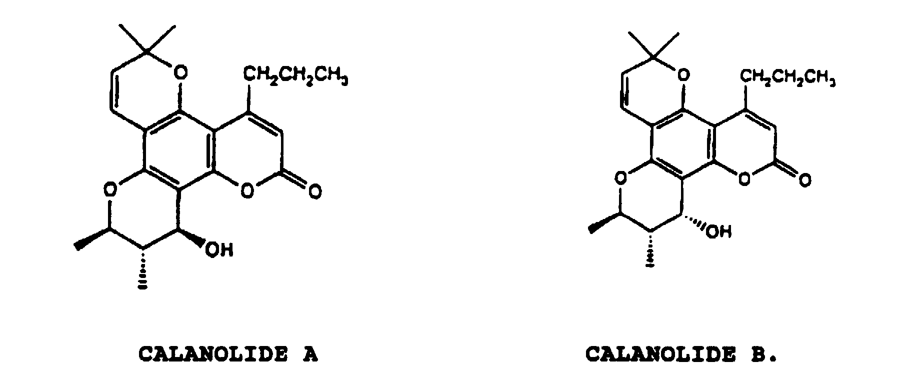 The antiviral calanolides and antiviral derivatives thereof may be described generically as having the structures below, wherein R¹ is C₁-C₆ alkyl or aryl; R² is 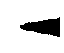 OH, 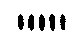 OH, 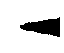 OR³, 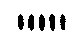 OR³, 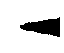 O₂CR³, 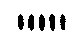 O₂CR³, 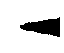 O₃SR³, or 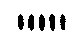 O₃SR³, wherein R³ is C₁-C₆ alkyl or aryl; R⁴ and R⁵ are the same or different and are each 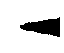 H, 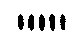 H, 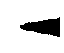 CH₃, or 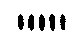 CH₃:

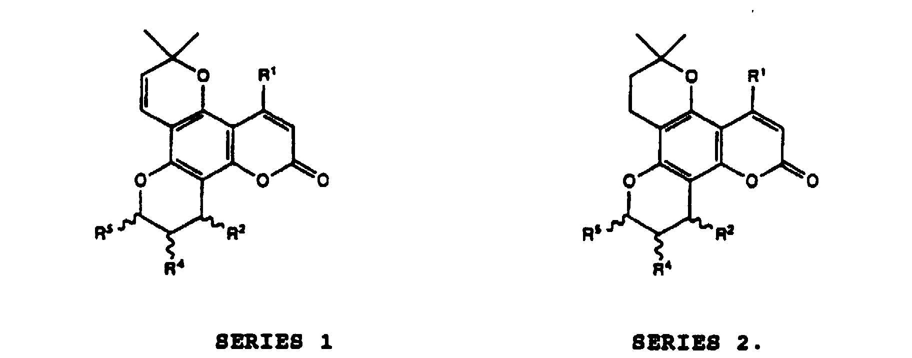

The present invention also provides a method of isolating and purifying calanolides and related derivatives from plants of the genus Calophyllum, in particular Calophyllum lanigerum, which comprises the steps of:
a) extracting dried plant material with organic solvents to obtain a crude extract;
b) solvent-solvent partitioning the crude extract to concentrate the antiviral compounds in a non-polar fraction;
c) subjecting the non-polar fraction to gel permeation or vacuum liquid chromatography; and,
d) isolating and purifying the calanolides by HPLC on silica gel and C₁₈ reversed phase columns.

The calanolides and derivatives thereof, obtained in accordance with the present inventive method, may be used alone or in combination with other antiviral agents in compositions, such as pharmaceutical compositions, to inhibit the growth or replication of a virus, such as a retrovirus, in particular a human immunodeficiency virus, specifically HIV-1 or HIV-2. It is expected that such compositions will have utility in the therapeutic treatment of an animal, in particular a human, infected with one or more of the above-cited viruses and in the prophylactic treatment of an animal, in particular a human, who is at risk for infection with one or more of the same viruses.

Prior to the discovery on which this invention is based, calanolides and derivatives thereof had not been isolated or described. Methods for isolating such compounds had not been determined. Accordingly, the antiviral activity of these compounds was not previously known, and the potential use of these compounds in compositions, such as pharmaceutical compositions, in the therapeutic and prophylactic treatment of viral infections in animals, in particular humans, had not been recognized.

The discovery that led to the present invention was the antiviral activity of an extract from Calophyllum lanigerum in an anti-HIV screen. The screen is one that has been operated by the U.S. National Cancer Institute since 1988 (see Boyd, M.R., in AIDS, Etiology, Diagnosis, Treatment and Prevention (DeVita V.T., Jr., Hellman S., Rosenberg S.A., eds.), pp. 305-319 (Philadelphia: Lippincott, 1988)).

Calophyllum lanigerum represents one of approximately 187 species of plants from the genus Calophyllum. A description of the plant can be found in: D.J. Mabberley, "The Plant Book," Cambridge University Press, 1987, p. 92. The genus is found principally in Indonesia, but is reported from Tahiti to East Africa. The plant material utilized in the examples herein was collected by J.S. Burley and B. Lee, under a U.S. National Cancer Institute contract to D. Soejarto, in Sarawak at an altitude of 3 meters and at a latitude of 2°N and a longitude of 110°E.

Previous phytochemical studies of the genus Calophyllum had revealed it to be a rich source of secondary metabolites. Xanthones (Dharmaratne, H.R.W., et al., Phytochemistry, 25: 1957-1959 (1986); Kumar, V., Phytochemistry, 21: 807-809 (1982); Somanathan, R., et al., J. Chem. Soc. Perkin I*,* 2515-1517 (1974)), steroids (Gunasekera, S.P., et al., J. Chem. Soc. Perkin I 2215-2220 (1975)), triterpenes (Gunatilaka, A.A.L., et al., Phytochemistry, 23: 323-328 (1984); Dahanayake, M., et al., J. Chem. Soc. Perkin I, 2510-2514 (1974)), coumarins (Samaraweera, U., et al., Tetrahedron Lett., 22: 5083-5086 (1981); Gautier, J., et al., Tetrahedron Lett., 27: 2715-2718 (1972)), and benzopyrans (Stout, G.H., J. Org. Chem., 33: 4185-4190 (1968)) are among the compounds reported from Calophyllum species; however, no antiviral activity had been previously associated with this genus.

A specific bioassay-guided strategy was used to isolate and purify the individual bioactive compounds from the extract of Calophyllum lanigerum. In this strategy, decisions concerning the overall chemical isolation method to be applied, and the nature of the individual steps therein, were determined by interpretation of biological testing data. The anti-HIV screening assay (Weislow, O.S., et al., J. Natl. Cancer Inst., 81(8): 577-586 (1989)) used in this process measured the degree of protection of human T-lymphoblastoid cells from the cytopathic effects of HIV. Fractions of the extracts were prepared using a variety of chemical means and were tested blindly in the primary screen. Active fractions were separated further, and the resulting subfractions were tested blindly in the screen. This process was repeated as many times as necessary in order to obtain the active, i.e., antiviral, fraction(s) representing pure compound(s), which then could be subjected to detailed chemical analysis and structure elucidation. In this manner, the new antiviral class of compounds described herein was discovered.

Although Calophyllum lanigerum was used as the principal source of calanolides in the present invention, it will be appreciated that such compounds also may be obtained from other species of the same genus.

A variety of methods can be used for the chemical isolation of calanolides. Among these methods are extraction, solvent-solvent partitioning, flash or vacuum liquid chromatography, gel permeation chromatography, and HPLC, with a variety of bonded phases. The isolation of the compounds can be monitored by UV, TLC, and anti-HIV bioassay. A typical isolation procedure is set forth below.

Approximately 0.2 kilogram of air-dried plant material, consisting of leaves, twigs, fruit or bark, is first ground to a fine powder and extracted with 1:1 MeOH-CH₂Cl₂; this is followed by a second extraction with methanol. These organic extracts typically amount to a total of about 5-20% of the mass of the starting plant material. The initial crude extract is dissolved in 4:1 MeOH-H₂O and extracted three times with CCl₄. The concentrated CCl₄ phase is fractionated by either gel permeation on Bio-Beads S-X4 (Bio-Rad Laboratories, Inc.; Richmond, CA) or vacuum liquid chromatography on silica gel. The calanolides are then purified from those column fractions that demonstrate HIV-inhibitory activity by sequential HPLC on silica (elution with 3:7 EtOAc-hexane) and C₁₈ reversed-phase (elution with 9:1 MeO₂H-H₂O) columns. Using this general procedure, either calanolide A or calanolide B, or both, can be obtained in an overall yield of about 0.05 - 0.2%; related compounds also present in the extract can be obtained in similar yields. The isolation of calanolides and derivatives thereof from C. lanigerum is described in greater detail in Example 1.

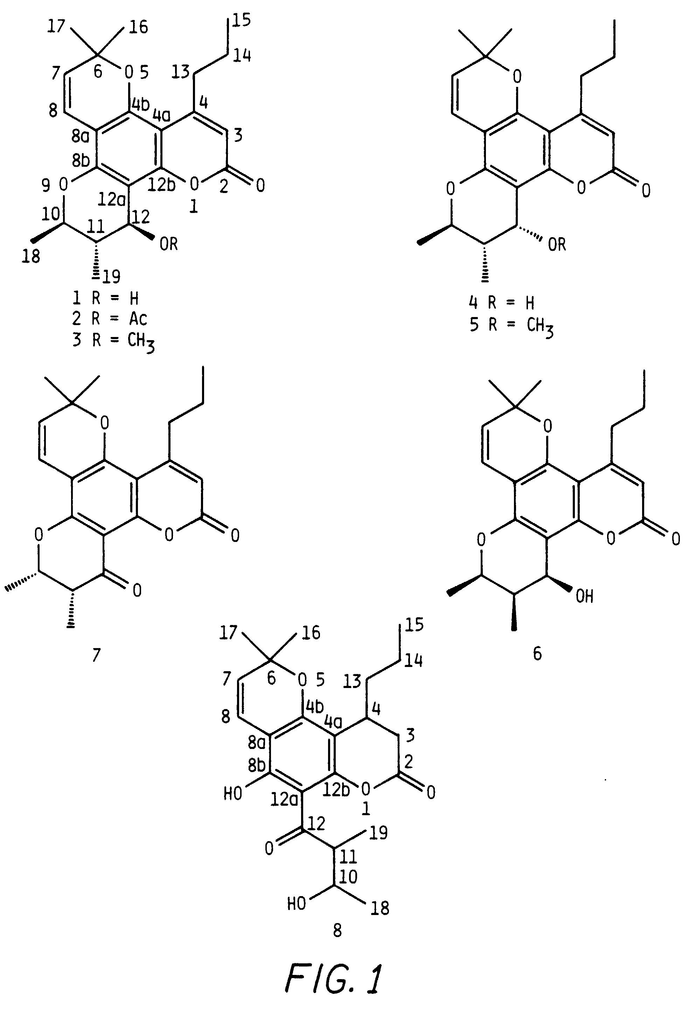

Chemical structures of calanolides and related derivatives, which can be isolated by the above general procedure from extracts of Calophyllum lanigerum are shown in Figure 1. The proofs of the structures can be obtained by a combination of methods, including primary spectral analyses (e.g., high-resolution NMR and mass spectrometry, infrared and UV spectroscopy), comparisons of spectral and physicochemical properties with related literature precedents, and by selected derivatization procedures, such as for determination of absolute stereochemistry. The structure proofs for calanolides A, B, and derivatives thereof from C. lanigerum are described in detail in Example 2 and summarized in Tables 1 and 2.

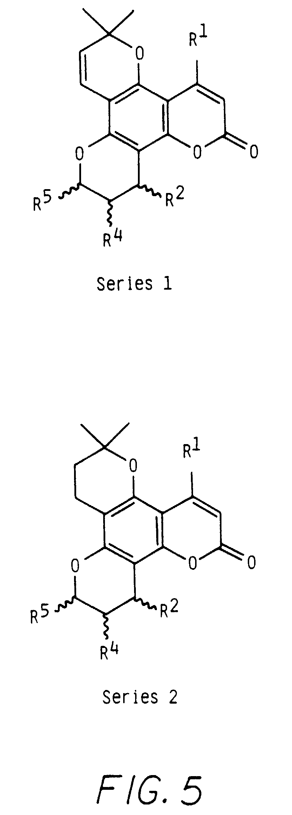

It will be appreciated by one who is skilled in the art that antiviral calanolides and derivatives thereof, other than those specifically described herein, may be isolated from other Calophyllum species and other natural sources and that such antiviral calanolides and derivatives thereof also may be synthesized chemically. Generic structural series are described in detail in Example 4 and Figure 5.

The antiviral activity of pure calanolides or derivatives thereof can be demonstrated further in a series of interrelated in vitro assays (Gulakowski, R.J., et al., J. Virol. Methods 33: 87-100 (1991)), which accurately predict antiviral activity of chemical compounds in humans. These assays measure the ability of compounds to prevent the replication of HIV and/or the cytopathic effects of HIV on human target cells. These measurements directly correlate with the pathogenesis of HIV-induced disease in vivo. Accordingly, the results of the analysis of the antiviral activity of calanolides and derivatives thereof from C. lanigerum, as set forth in Example 3 and as illustrated in Figure 4 A-D, are believed to predict accurately the antiviral activity of these compounds in humans.

The compounds of the present invention can be shown to inhibit a retrovirus, such as the human immunodeficiency virus, specifically HIV-1 and HIV-2. As one skilled in the art will appreciate, the compounds of the present invention probably will inhibit other retroviruses and may inhibit viruses, other than retroviruses. Examples of viruses that may be treated in accordance with the present invention include, but are not limited to, Type C and Type D retroviruses, HTLV-1, HTLV-2, HIV, FLV, SIV, MLV, BLV, BIV, equine infectious, anemia virus, avian sarcoma viruses, such as rous sarcoma virus (RSV), hepatitis type A, B, non-A, and non-B viruses, herpes viruses, cytomegaloviruses, influenza viruses, arboviruses, varicella viruses, measles, mumps and rubella viruses.

The antiviral calanolides and antiviral derivatives thereof may be formulated into various compositions for use in therapeutic and prophylactic treatment methods. The present inventive composition may be used to treat a virally infected animal, such as a human. The compositions of the present invention are particularly useful in inhibiting the growth or replication of a virus, such as a retrovirus, in particular a human immunodeficiency virus, specifically HIV-1 and HIV-2. The compositions also are expected to be useful in the treatment of animals, such as humans, infected with other viruses, such as those listed above. Furthermore, such compositions should find utility in the prophylactic treatment of animals, such as humans, who are at risk for viral infection.

Compositions for use in the prophylactic or therapeutic treatment methods of the present invention comprise one or more antiviral calanolides or antiviral derivatives thereof and a pharmaceutically acceptable carrier. Pharmaceutically acceptable carriers are well-known to those who are skilled in the art, as are suitable methods of administration. The choice of carrier will be determined in part by the particular calanolide compound, as well as by the particular method used to administer the composition.

One skilled in the art will appreciate that various routes of administering a drug are available and, although more than one route may be used to administer a particular drug, a particular route may provide a more immediate and more effective reaction than another route. Furthermore, one skilled in the art will appreciate that the particular pharmaceutical carrier employed will depend, in part, upon the particular compound employed and the chosen route of administration. Accordingly, there is a wide variety of suitable formulations of the composition of the present invention.

Formulations suitable for oral administration may consist of liquid solutions, such as an effective amount of the compound dissolved in diluents, such as water, saline, or orange juice; capsules, sachets or tablets, each containing a predetermined amount of the active ingredient, as solids or granules; solutions or suspensions in an aqueous liquid; and oil-in-water emulsions or water-in-oil emulsions. Tablet forms may include one or more of lactose, mannitol, corn starch, potato starch, microcrystalline cellulose, acacia, gelatin, colloidal silicon dioxide, croscarmeliose sodium, talc, magnesium stearate, stearic acid, and other excipients, colorants, diluents, buffering agents, moistening agents, preservatives, flavoring agents, and pharmacologically compatible carriers.

The antiviral calanolides or antiviral derivatives thereof, alone or in combination with other antiviral compounds, can be made into aerosol formulations to be administered via inhalation. These aerosol formulations can be placed into pressurized acceptable propellants, such as dichlorodifluoromethane, propane, nitrogen, and the like.

Formulations suitable for topical administration include lozenges comprising the active ingredient in a flavor, usually sucrose and acacia or tragacanth; pastilles comprising the active ingredient in an inert base, such as gelatin and glycerin, or sucrose and acacia; and mouthwashes comprising the active ingredient in a suitable liquid carrier; as well as creams, emulsions, gels, and the like containing, in addition to the active ingredient, such carriers as are known in the art.

Formulations for rectal administration may be presented as a suppository with a suitable base comprising, for example, cocoa butter or a salicylate.

Formulations suitable for vaginal administration may be presented as pessaries, tampons, creams, gels, pastes, foams, or spray formulas containing, in addition to the active ingredient, such carriers as are known in the art to be appropriate. Similarly, the active ingredient may be combined with a lubricant as a coating on a condom.

Formulations suitable for parenteral administration include aqueous and non-aqueous, isotonic sterile injection solutions, which may contain anti-oxidants, buffers, bacteriostats, and solutes that render the formulation isotonic with the blood of the intended recipient, and aqueous and non-aqueous sterile suspensions that may include suspending agents, solubilizers, thickening agents, stabilizers, and preservatives. The formulations may be presented in unit-dose or multi-dose sealed containers, such as ampules and vials, and may be stored in a freeze-dried (lyophilized) condition requiring only the addition of the sterile liquid carrier, for example, water, for injections, immediately prior to use. Extemporaneous injection solutions and suspensions may be prepared from sterile powders, granules, and tablets of the kind previously described.

The dose administered to an animal, particularly a human, in the context of the present invention should be sufficient to effect a prophylactic or therapeutic response in the infected individual over a reasonable time frame. The dose will be determined by the strength of the particular calanolide compound employed, the severity of the disease state, as well as the body weight and age of the infected individual. The size of the dose also will be determined by the existence of any adverse side effects that may accompany the particular compound employed. It is always desirable, whenever possible, to keep adverse side effects to a minimum.

The dosage may be in unit dosage form, such as a tablet or capsule. The term "unit dosage form" as used herein refers to physically discrete units suitable as unitary dosages for human and animal subjects, each unit containing a predetermined quantity of calanolides or derivatives thereof, alone or in combination with other antiviral agents, calculated in an amount sufficient to produce the desired effect in association with a pharmaceutically acceptable diluent, carrier, or vehicle.

The specifications for the unit dosage forms of the present invention depend on the particular compound or compounds employed and the effect to be achieved, as well as the pharmacodynamics associated with each compound in the host. The dose administered should be an "antiviral effective amount" or an amount necessary to achieve an "effective level" in the individual patient.

Since the "effective level" is used as the preferred endpoint for dosing, the actual dose and schedule may vary, depending upon interindividual differences in pharmacokinetics, drug distribution, and metabolism. The "effective level" may be defined, for example, as the blood or tissue level desired in the patient that corresponds to a concentration of one or more calanolides, or derivatives thereof, which inhibits a virus such as HIV in an assay known to predict for clinical antiviral activity of chemical compounds. The "effective level" for compounds of the present invention also may vary when the compositions of the present invention are used in combination with AZT or other known antiviral compounds or combinations thereof.

One skilled in the art can easily determine the appropriate dose, schedule, and method of administration for the exact formulation of the composition being used, in order to achieve the desired "effective concentration" in the individual patient. One skilled in the art also can readily determine and use an appropriate indicator of the "effective concentration" of the compounds of the present invention by a direct (e.g., analytical chemical analysis) or indirect (e.g., with surrogate indicators such as p24 or RT) analysis of appropriate patient samples (e.g., blood and/or tissues).

In the treatment of some virally infected individuals, it may be desirable to utilize a "mega-dosing" regimen, wherein a large dose of the calanolide or derivative thereof is administered, time is allowed for the compound to act, and then a suitable reagent is administered to the individual to inactivate the calanolide or derivative thereof.

The pharmaceutical composition may contain other pharmaceuticals, in conjunction with the calanolide or derivative thereof, when used to therapeutically treat acquired immundoeficiency syndrome (AIDS). Representative examples of these additional pharmaceuticals include antiviral compounds, immunomodulators, immunostimulants, and antibiotics. Exemplary antiviral compounds include AZT, ddI, ddC, gancyclovir, fluorinated dideoxynucleotides, nonnucleoside analog compounds such as nevirapine (Shih et al. PNAS 88:9878-9882 (1991)), TIBO derivatives such as R82913 (White et al., Antiviral Research 16:257-266 (1991)), Ro 31-8959 (Craig et al., Antiviral Research 16:295-305 (1991)), BI-RJ-70 (Shih et al.), acyclovir, α-interferon and recombinant CD4 (Merigan et al., The American Journal of Medicine 90 (Suppl. 4A): 8S-17S (1991)). Exemplary immunomodulators and immunostimulants include various interleukins, CD4, cytokines, antibody preparations, blood transfusions, and cell transfusions. Exemplary antibiotics include antifungal agents, antibacterial agents, and anti-Pneumocystis carinii agents.

Administration of the inhibitory compound with other anti-retroviral agents and particularly with known reverse transcriptase (RT) inhibitors, such as ddC, AZT, ddI, ddA, or other inhibitors that act against other HIV proteins, such as anti-TAT agents, will generally inhibit most or all replicative stages of the viral life cycle. The dosages of ddC and AZT used in AIDS or ARC patients have been published. A virustatic range of ddC is generally between 0.05 µM to 1.0 µM. A range of about 0.005-0.25 mg/kg body weight is virustatic in most patients. The preliminary dose ranges for oral administration are somewhat broader, for example 0.001 to 0.25 mg/kg given in one or more doses at intervals of 2, 4, 6, 8, 12, etc. hours. Currently 0.01 mg/kg body weight ddC given every 8 hours is preferred. When given in combined therapy, the other antiviral compound, for example, may be given at the same time as the calanolide compound or the dosing may be staggered as desired. The two drugs also may be combined in a composition. Doses of each may be less when used in combination than when either is used alone.

The present inventive compounds and methods are further described in the context of the following examples. These examples serve to illustrate further the present invention and are not intended to limit the scope of the invention.

### Example 1

This example illustrates the isolation of calanolides and derivatives thereof from extracts of Calophyllum lanigerum. Dried fruit and twigs (763 g) of Calophyllum lanigerum were stored at -20° until the material was ground, percolated in 1:1 CH₂Cl₂/MeOH, and washed with 100% MeOH. Removal of the solvent under reduced pressure provided 72.5 g of organic extract.

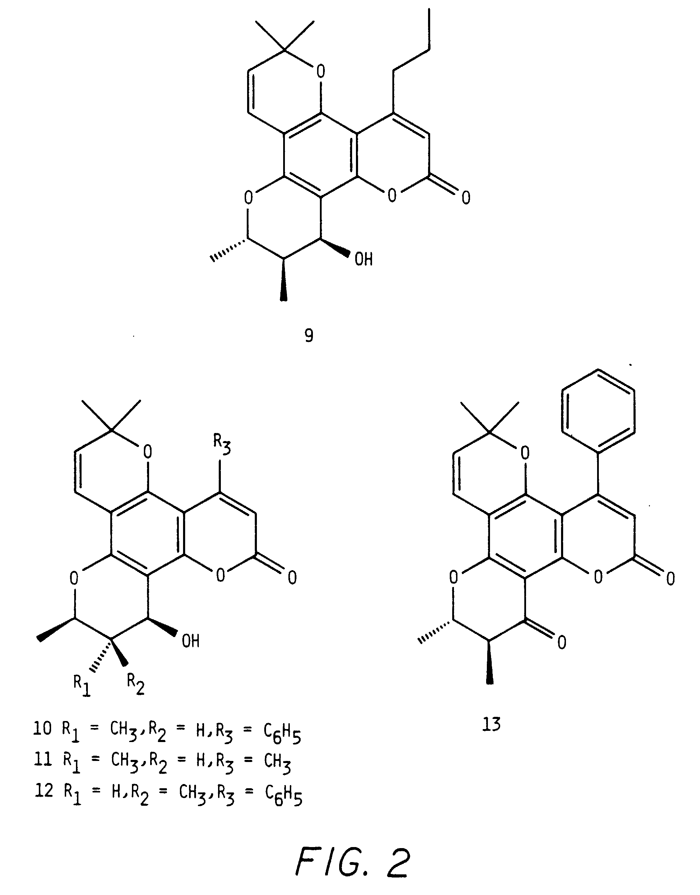

A 10 g portion of the organic extract was subjected to a solvent-solvent partitioning protocol. A 10g portion of the organic extract was suspended in 500 ml CH₃OH-H₂O (9:1) and extracted with hexane (3 x 300 ml). The water content of the polar phase was increased to 20%, whereupon a second extraction was conducted with CCl₄ (3 x 300 ml). The anti-HIV activity of all fractions was assessed using a routine primary screening assay (Weislow, O.S., et al., J. Natl. Cancer Inst., 81(8): 577-586 (1989)) at each chromatographic step. Anti-HIV activity was concentrated in the hexane (770 mg) and CCl₄ (590 mg) soluble fractions. The active fractions were individually separated by vacuum liquid chromatography (VLC) on 10 g of silica gel using mixtures of hexane/EtOAc. The active constituents that eluted with 10-25% EtOAc were combined, based upon TLC and ¹H NMR profiles, to provide two active fractions. The individual fractions were further separated by VLC on silica, using gradual step-gradient elution with hexane/EtOAc mixtures. Final purification gave eight compounds (**1-8**), the structures of which are given in Figure 1. Silica HPLC eluted with 7:3 hexane/EtOAc gave calanolide A (**1**) (11.7 mg), calanolide B (**4**) (5.0 mg), and compound **7** (12.5 mg). Purification of the other components was effected by C₁₈ HPLC with 9:1 MeOH/H₂O to give 12-acetoxycalanolide A (**2**) (7 mg), 12-methoxycalanolide A (**3**) (5 mg), 12-methoxycalanolide B (**5**) (16 mg), compound **6** (4 mg), and compound 8 (11 mg). Figure 2 shows five related structures (**9-13**) previously reported from other sources. By comparing the novel compounds of Figure 1 with the known compounds of Figure 2, it can be seen that the compounds of the present invention differ from those compounds previously reported for other sources.

### Example 2

This example sets out the structure proofs for calanolides A and B and related derivatives from Calophyllum lanigerum, which were obtained in Example 1. Calanolide A (**1**) was isolated as an optically active oil, [α]_{D} = +60°, which gave a HREIMS parent ion at *m/z* 370.1736 daltons, indicating a molecular formula of C₂₂H₂₆O₅. The mass spectrum contained significant fragment ions for M⁺-CH₃ (*m/z* 355, 100%), M⁺-CH₃-H₂O (*m/z* 337, 12%) and M⁺-C₅H₁₁ (*m/z* 299, 29%). The infrared spectrum showed bands corresponding to hydroxyl (3300 cm⁻¹) and carbonyl (1735 cm⁻¹) groups. Resonances for 11 sp² carbons in the ¹³C NMR spectrum revealed a conjugated ester (δ 160.4), a disubstituted olefin conjugated to a phenyl group (δ 126.9 [1H] and 116.5 [1H]), a trisubstituted olefin conjugated to a carbonyl (δ 158.9 and 110.1 [1H]), and a fully substituted benzene ring bearing three oxygen moieties (δ 154.5, 153.1, 151.1, 106.3, 106.4, and 104.0). Taking into account the number of double bond equivalents implicit in the molecular formula, calanolide A (**1**) was determined to be tetracyclic. The ¹H NMR spectrum showed two methyl singlets (δ 1.49 and 1.44), two methyl doublets (δ 1.44 and 1.13), and a methyl triplet (δ 1.01). Additional proton signals included those of an allylic methylene (δ 2.87 [2H], m), an aliphatic methylene (1.63 [2H], m), and three olefin protons (δ 6.60 d, J = 9.5 Hz; 5.92 t, J = 1.0 Hz; 5.52 d, J = 9.5 Hz). These data suggested that calanolide A (**1**) was a coumarin derivative related to costatolide (**9**), a metabolite previously reported from Calophyllum costatum (Stout, G.H., J. Org. Chem., 29: 3604-3609 (1964)) .

One-bond and long-range proton detected heteronuclear correlation experiments (HMQC and HMBC) allowed the complete assignment of both the ¹H NMR (Table 1) and ¹³C NMR spectra (Table 2) of calanolide A (**1**). Key correlations included those between H8 and carbons 4b, 6, 8a, and 8b, which helped to establish the position of the 2,2-dimethylchromene system. Placement of the n-propyl group at C4 was aided by a 1.0 Hz allylic coupling between the C13 allylic methylene protons and the C3 olefin proton, and by three-bond heteronuclear correlations from the C13 methylene protons to C3 and C4a. The remaining substitution pattern about the coumarin nucleus was defined by correlations between H12 and carbons 8b, 10, 11, 12a, 12b, and 19. This confirmed that, although calanolide A (**1**) had the same skeleton as costatolide (**9**), they differed in the relative stereochemistry of their substituents about the 2,3-dimethylchromanol ring.

In the ¹H NMR spectrum of compound **1**, the H12 benzylic carbinol proton showed a 8.0 Hz coupling to H11, which revealed that these two protons had a trans-diaxial orientation. A 9.0 Hz coupling between Hll and H10 established that H10 also was axial. This assignment was supported by nOe enhancements (3%) observed between the diaxial H10 and H12 protons. Calanolide A (**1**) was thus determined to be the C12 epimer of costatolide (**9**), which showed J₁₀₋₁₁ and J₁₁₋₁₂ of 10.5 Hz and 3.5 Hz, respectively (Stout, G.H., et al., J. Org. Chem. 29: 3604-3609 (1964)). Two related coumarin derivatives, inophyllum B (**10**) (Bandara, B.M.R., et al., Phytochemistry, 25(2): 425-428 (1986)) and cordatolide A (**11**) (Dharmaratne, H.R.W., et al., Phytochemistry 24: 1553-1557 (1985)) reportedly have the same relative stereochemical features about the chromanol ring as those found in compound 1, but differ in their C4 substituents. The J₁₀₋₁₁ and J₁₁₋₁₂ coupling constants observed in calanolide A (1) agree closely with those described in the above-cited publications for compounds **10** and **11**.

Other physicochemical and spectral data for calanolide A (**1**) were as follows: [α]_{D} + 60° (CHCl₃, c 0.7); UV λₘₐₓ (MeOH) 325 (ε 13,700), 284 (ε 22,800), 228 (ε 22,200) nm; IR (film) νₘₐₓ 3439, 2966, 1735, 1713, 1582, 1111 cm⁻¹; HREIMS obs. m/z 370.1764, calc'd for C₂₂H₂₆O₅, 370.1780; low res. MS m/z 370 (38%), 355 (100%), 337 (12%), 299 (29%).

12-Acetoxycalanolide A (**2**), [α]_{D}= +20°, gave a parent ion by HREIMS at m/z 412.1825 daltons corresponding to a molecular formula of C₂₄H₂₈O₆. Significant fragment ions were observed for M⁺-CH₃ (*m/z* 397, 41%), M⁺-AcOH (m/z 352, 30%) and M⁺-AcOH-CH₃ (*m/z* 337, 100%). The presence of an acetate group was suggested by a sharp 3H singlet in the ¹H NMR spectrum at δ 2.10 and ¹³C NMR resonances at δ 21.2 (3H) and 170.7. The remaining ¹H and ¹³C NMR signals for compound **2** were very similar to those recorded for calanolide A (**1**), except that the H12 resonance in compound **2** was shifted downfield to δ 5.97. This suggested that compound **2** was the 12-acetoxy derivative of calanolide A (**1**). The J₁₀₋₁₁ (6.5 Hz) and J₁₁₋₁₂ (6.0 Hz) couplings in compound **2** supported a pseudoaxial orientation of the chromanol ring protons. The slightly diminished chromanol proton couplings in compound **2** conceivably resulted from a slight twisting of the flexible chromanol ring. Further evidence for the proposed substituent configuration was provided by difference nOe enhancements of 2% measured between H10 and H12.

Other physicochemical and spectral data for compound **2** were as follows: [α]_{D} + 20° (CHCl₃, c 0.6); IR (film) λₘₐₓ 2960, 1738, 1585, 1376, 1230, 1138 cm⁻¹; HREIMS obs. m/z 412.1825, calc'd for C₂₄H₂₈O₆, 412.1886; low res. MS m/z 412 (13%), 397 (41%), 352 (30%), 337 (100%), 299 (8%).

The HREIMS of 12-methoxycalanolide A (**3**), [α]_{D}= +32°, showed a parent ion at *m/z* 384.1924 daltons, corresponding to a molecular formula of C₂₃H₂₈O₅. Significant fragment ions observed for M⁺-CH₃ (m/z 369, 12%), M⁺-CH₃OH (*m/z* 352, 9%) and M⁺-CH₃OH-CH₃ (*m/z* 337, 100%) suggested the presence of a methoxyl group, which was confirmed by a ¹H NMR singlet (δ 3.59, 3H) and a corresponding carbon resonance at δ 57.6. The ¹H and ¹³C NMR spectra revealed that compound 3 had the same skeleton as calanolide A (**1**). However, important differences were observed in the signals for some of the chromanol ring substituents. In addition to the vicinal couplings of J₁₀₋₁₁ (3.5 Hz) and J₁₁₋₁₂ (3.7 Hz), a W coupling of 1.3 Hz was observed between H10 and H12 in compound **3**. The W coupling required a pseudodiequatorial configuration for the C10 and C12 protons. Significant nOe enhancements between H11 and both the C10 methyl group (3.5%) and the C12 methoxyl group (3.5%) indicated that H11 was cis to these two substituents and, therefore, had an equatorial orientation about the chromanol ring. It appeared that, in 12-methoxycalanolide A (**3**), the preferred conformation of the chromanol ring was inverted relative to calanolide A (**1**). Thus, while H10, H11, and H12 were oriented α, β, α respectively in both compounds, in calanolide A (**1**) all three protons were axial, and in 12-methoxycalanolide A (**3**) they were all equatorial.

Other physicochemical and spectral data for compound **3** were as follows: [α]_{D} + 32° (CHCl₃, c 0.8); IR (film) λₘₐₓ 2960, 1731, 1584, 1380, 1137, cm⁻¹; HREIMS obs. m/z 384.1924, calc'd for C₂₃H₂₈O₅, 384.1937; low res. MS m/z 384 (5%), 369 (12%), 352 (9%), 337 (100%).

Calanolide B (**4**), [α]_{D}= +8°, was isomeric to calanolide A (**1**), as it also showed a HREIMS parent ion at *m/z* 370.1747 daltons, corresponding to C₂₂H₂₆O₅. The ¹H and ¹³C NMR spectra of calanolide B (**4**) were virtually identical to those from calanolide A (**1**), with the exception of some variations in signals from the chromanol ring. It was clear from the spectral data that compound **4** differed from compound **1** only in the stereochemical disposition of the chromanol ring substituents. Proton-proton coupling constant analysis showed a 10.5 Hz J₁₀₋₁₁ coupling and a 3.3 Hz J₁₁₋₁₂ coupling. Thus, H10 and H11 were trans-diaxial while H11 and H12 were in a cis configuration with H12 in a pseudoequatorial orientation. Calanolide B (**4**) had the same relative stereochemistry as costatolide (**9**) but its optical rotation was opposite in sign to that reported for compound **9** (Stout, G.H., et al., J. Org. Chem., 29: 3604-3609 (1964)); therefore, compounds **4** and **9** are enantiomeric.

Other physicochemical and spectral data for calanolide B (**4**) were as follows: [α]_{D} + 10° (acetone, c 1.0) UV λₘₐₓ (MeOH) 325 (ε 13,700), 284 (ε 22,800), 228 (ε 22,200) nm; IR (film) νₘₐₓ 3470, 2970, 1732, 1640, 1608, 1587 cm⁻¹; HREIMS obs. m/z 370.1747, calc'd for C₂₂H₂₆O₅, 370.1780; low res. MS m/z 370 (3%), 355 (100%), 337 (13%), 300 (5%), 299 (20%).

12-Methoxycalanolide B (**5**), [α]_{D}= +34°, provided a HREIMS parent ion at *m/z* 384.1890 daltons appropriate for a molecular formula of C₂₃H₂₈O₅. Additional fragment ions were seen for M⁺-CH₃ (*m/z* 369, 12 %), M⁺-CH₃OH (*m/z* 352, 13%) and M⁺-CH₃OH-CH₃ (*m/z* 337, 100%). The ¹H and ¹³C NMR spectra of compound **5** were virtually identical to those recorded for compound **4**, with the addition of a sharp 3H singlet at δ 3.58 and a corresponding carbon resonance at δ 59.4. These data indicated that compound **5** was the 12-methoxyl derivative of calanolide B (4). This assignment was confirmed by acid hydrolysis of 2 mg of compound 5 in 400 µl of THF/H₂O and 8 µl of 6N HCl at room temperature for 48 hr to provide compound **4** as the only product.

Other physicochemical and spectral data for compound 5 were as follows: [α]_{D} + 34° (CHCl₃, c 0.5); IR (film) λₘₐₓ 2966, 1734, 1716, 1700, 1558, 1540, 1506, 1457 cm⁻¹; HREIMS obs. m/z 384.1890, calc'd for C₂₃H₂₈O₅, 384.1937; low res. MS m/z 384 (4%), 369 (12%), 352 (13%), 337 (100%).

Compound **6**, [α]_{D}= +68°, also was isomeric with calanolide A (**1**), since it showed similarly a HREIMS parent ion at *m/z* 370.1695 daltons, consistent with a molecular formula of C₂₂H₂₆O₅. Fragment ions were found at M⁺-CH₃ (*m/z* 355, 100%), M⁺-CH₃-H₂O (*m/z* 337, 25%) and M⁺-C₅H₁₁ (*m/z* 299, 35%). Again, the only notable differences between the ¹H and ¹³C NMR spectra of **6** and those recorded for compound **1** were the resonances associated with the chromanol ring. The J₁₀₋₁₁ in compound **6** was 2.5 Hz, while J₁₁₋₁₂ was 6.0 Hz. These coupling constants were insufficient to define the relative stereochemistry of carbons 10, 11, and 12. However, the C12 hydroxyl proton gave a sharpened peak with a 1.5 Hz coupling to H12 which suggested that the rate of exchange of the OH proton was reduced due to hydrogen bonding to O1. Hydrogen bonding to O1 would require an equatorial OH at C12. A 5% nOe enhancement between H10 and H12 confirmed that these protons each had axial orientations. Therefore, H11 had to be equatorial. Compound **6** was thus the C11 epimer of calanolide A (**1**) and had the same substitution pattern and relative stereochemistry about the chromanol ring as the previously described coumarin derivative inophyllum A (**12**) (Bandara, B.M.R., et al., Phytochemistry 25: 425-428 (1986)); the J₁₀₋₁₁ (3.3 Hz) and J₁₁₋₁₂ values (5.4 Hz) reported earlier for compound **12** were in good agreement with the respective couplings observed in compound **6**.

Other physicochemical and spectral data for compound **6** were as follows: [α]_{D} + 68° (CHCl₃, c 0.7); IR (film) λₘₐₓ 2960, 1729, 1620, 1582, 1120 cm⁻¹; HREIMS obs. m/z 370.1695, calc'd for C₂₂H₂₆O₅, 370.1780; low res. MS m/z 370 (52%), 355 (100%), 337 (25%), 200 (35%).

Compound **7**, [α]_{D}= +60°, provided a HREIMS molecular ion at *m/z* 353.1352 appropriate for a molecular formula of C₂₂H₂₄O₅. This required that compound 7 had one additional unsaturation equivalent relative to calanolide A (1). The infrared spectrum, with bands at 1734 and 1697 cm⁻¹, suggested the presence of an additional carbonyl group. Heteronuclear correlation experiments allowed the complete assignment of the ¹H and ¹³C NMR spectra of compound **7**. While the ¹³C NMR spectrum of compound **7** was quite similar to that of calanolide A (**1**), the C12 peak in compound **7** was shifted downfield to δ 192.9, indicative of an α,β-unsaturated ketone functionality. A shift of the C11 proton resonance in compound 7 to δ 2.61 supported its placement α to a ketone carbonyl.

The small coupling measured between H10 and H11 (J₁₀₋₁₁ = 3.0 Hz) indicated that at least one of these protons was equatorial. The previously described oxidation product (**13**) of soulattrolide (Gunasekera, S.P., et al., J. Chem. Soc., 1505-1511 (1977)) contains a similar 2,3-dimethylbenzopyranone ring system. In compound **13**, H10 and H1 are trans, and a J₁₀₋₁₁ coupling of 11.0 Hz was reported. This indicated that when the H10 and H11 protons were trans, the ring adopted a conformation with these two protons in a diaxial orientation. Therefore, the relative stereochemistry of the H10 and H11 protons in compound 7 had to be cis. The absolute stereochemistry at C10 and C11 has not been determined and, therefore, both of the corresponding methyls have been drawn arbitrarily as a in Figure 1.

Other physicochemical and spectral data for compound **7** were as follows: [α]_{D} + 60° (CHCl₃, c 0.5); IR (film) λₘₐₓ 2960, 1734, 1697, 1684, 1575, 1558 cm⁻¹; HREIMS obs. m/z 368.1213, calc'd for C₂₂H₂₄O₅, 368.1624; low res. MS m/z 368 (25%), 353 (100%), 297 (68%).

Compound **8**, [α]_{D}= +30°, had a molecular formula of C₂₂H₂₈O₆, as indicated by the HREIMS parent ion at *m/z* 388.1890 daltons. Fragment ions appropriate for M⁺-CH₃ (*m/z* 373, 100%), M⁺-C₃H₇ (*m/z* 345, 3%), M⁺-CO₂CH₃ (*m/z* 329, 5%), M⁺-C₃H₇O₂ (*m/z* 313, 3%) and M⁺-COCHCH₃CHOHCH₃ (*m/z* 287, 3%) were also observed. The complete ¹H and ¹³C NMR spectra of compound 8 were assigned with information provided from nOe experiments and heteronuclear correlations. The ¹³C NMR spectrum contained signals for an unsaturated ketone (δ 201.0), a saturated ester (δ 178.6), a disubstituted olefin (δ 125.6 [1H] and 115.6 [1H]) and a fully substituted benzene ring bearing three oxygens (δ 160.0 [2C], 157.3, 108.9, 102.6 and 101.2). Therefore, compound **8** had only three of the four rings found in the other members of the calanolide series. In contrast to compounds **1-7**, which gave vivid blue spots on TLC when charred with vanillin/H₂SO₄, compound **8** gave a brownish-green spot.

The ¹H and ¹³C NMR spectra of compound **8** showed some resonances that corresponded closely to the coumarin and 2,2-dimethylchromene ring systems of compounds **1-7**. However, the C3/C4 double bond in compounds **1-7** was fully saturated in compound **8**. For clarity of discussion, as depicted in Figure 2, the carbon atoms have been numbered in compound 8 in a scheme that is analogous to the numbering shown for calanolide A (**1**) and which likewise applies to the others of the series, i.e., compounds **2-7**. The saturation of the double bond resulted in a methylene (δ 2.81 dd, J = 15.0, 9.0 Hz and 2.67 dd, J = 15.0, 6.5 Hz) α to the lactone carbonyl that was coupled to the C4 benzylic methine (δ 3.67 m). The C4 proton also showed heteronuclear correlations to C2, C3, C4a, C12b, C13 and C14, which supported the presence of a 3,4-dihydrocoumarin skeleton with an n-propyl substituent at C4. Heteronuclear correlations, including those between H8 and C4b, C6, and C8b, confirmed the placement of the chromene functionality on the coumarin ring system. This suggested that the chromanol ring system present in compounds **1-7** was open in compound **8**.

The position of the phenol on C8b was established by heteronuclear correlations from the phenolic proton to C8a, C8b, and C12a and an nOe interaction with H8. Since the remaining ketone group in the molecule was unsaturated, it had to be located on C12. In this position it could accept a hydrogen bond from the C8b phenol proton, which appeared as a sharp singlet at δ 12.40. The downfield shift of H11 (δ 2.52 dq, J = 3.5, 6.5 Hz) was appropriate for a methine located α to a ketone. The C10 carbinol methine proton (6 4.49 dq, J = 7.0, 3.5 Hz) showed vicinal coupling to H11 and to the C18 methyl group. All heteronuclear correlations, including those from the C19 methyl protons to C10, C11 and C12, were fully consistent with the proposed structure for compound **8**.

Other physicochemical and spectral data for compound **8** (Figure 1) were as follows: [α]_{D} + 30° (CHCl₃, c 0.5); IR (film) λₘₐₓ 2960, 1706, 1644, 1625, 1442, 1131 cm⁻¹; HREIMS obs. m/z 388.1890, calc'd for C₂₂H₂₈O₆, 388.1886; low res. MS m/z 388 (19%), 373 (100%), 345 (3%), 329 (5%), 313 (3%), 287 (3%).

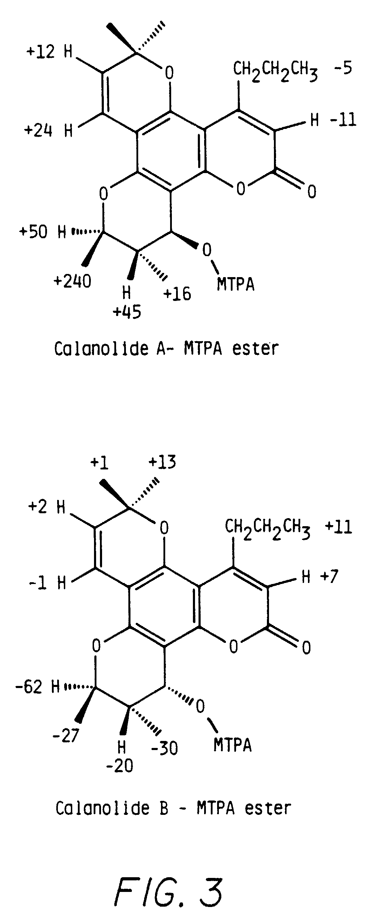

The absolute stereochemistry of calanolide A (1) and calanolide B (**4**) was determined by a modified Mosher's method (Ohtani, I., et al., Tetrahedron Lett., 30(24): 3147-3150 (1989); J. Org. Chem., 56: 1296-1298 (1991)). The technique utilized anisotropic shifts induced in the ¹H NMR spectra of methoxy(trifluoromethyl)-phenylacetic (MTPA) esters of secondary alcohols to define the absolute stereochemistry. Both (+)-(R)- and (-)-(S)-MTPA esters (Figure 3) of compounds **1** and **4** were prepared. A solution of (R)-methoxy (trifluoromethyl)-phenylacetic acid chloride (2.5 mg in 50 µl of benzene) was added to 3 mg of calanolide A (**1**) dissolved in 3 ml of dry benzene. A 0.03 mg aliquot of dimethylaminopyridine and 10 µl of triethylamine were added and the reaction mixture was refluxed. After 3 hours, a second 2.5 mg portion of (R)-MTPA-chloride was added, and the reaction was refluxed for an additional 21 hours. When the mixture had cooled, 10 ml of benzene were added and the organic phase was successively washed with 10% HCl, 1N NaHCO₃, and H₂O. The solution was dried over Na₂SO₄, evaporated to dryness, and then quickly chromatographed on a short plug (1 cm x 2 cm) of silica, eluting with mixtures of hexane/EtOAc. A compound, which appeared to be an elimination product, eluted first with 5% EtOAc, while the desired (R)-MTPA ester eluted with 12% EtOAc. The same procedure was repeated with (S)-MTPA-chloride to give the (S)-MTPA ester. The (R)- and (S)-MTPA esters of calanolide B (2) were prepared in an identical fashion, with the exception that, after the second addition of the MTPA-chloride, the reaction mixture was refluxed for only 2 additional hours (total time of reflux = 5 hr). The Δδ values (Figure 3) from the 500 MHz ¹H NMR spectra were calculated (Δδ = δₛ-δ_{R}). By this method, the absolute configuration of C12 was determined to be 12S in calanolide A (**1**) and 12R in calanolide B (**4**). As established earlier, calanolide A (1) [10R, 11R, 12S] and calanolide B (**4**) [10R, 11R, 12R] were C12 epimers.

Esterification of calanolide A (**1**) occurred slowly (24 hr reflux for compound **1** vs. 5 hr for compound **4**), and, by ¹H NMR analysis, esterification appeared to cause a change in the conformation of the chromanol ring. The methyls and the ester group flipped from equatorial to axial positions in the MTPA ester of compound **1,** as J₁₀₋₁₁= 2.5 Hz (previously 9.0 Hz) and J₁₁₋₁₂= 2.5 Hz (previously 8.0 Hz). In addition, a 4-bond W coupling of 1.5 Hz between H10 and H12 also could be observed. Similar changes in the conformation of the chromanol ring were previously noted for compound 3. The anisotropic shifts induced in the MTPA esters indicated that the bulky MTPA group was sterically repulsed by the coumarin ring lactone. Thus, the plane that divided the molecule's proton resonances into Δδ-positive and Δδ-negative did not cleanly bisect the dihydropyran ring through C12 and O9. In calanolide A (**1**), the dividing plane seemed to come closer to C6 and, in calanolide B (**4**), to C8b. Due to the 1,3-diaxial orientation of the C10β-methyl and the C12β-ester group in the MTPA ester of compound **1**, the former methyl group was very strongly influenced by the ester (Δδ = +240 in comparison to +16 measured for the C11α-methyl).

Summaries of the 500 MHz ¹H-NMR data and the 125 MHz ¹³C-NMR data for compounds **1-8** (Figure 1) are provided in Tables 1 and 2, respectively.

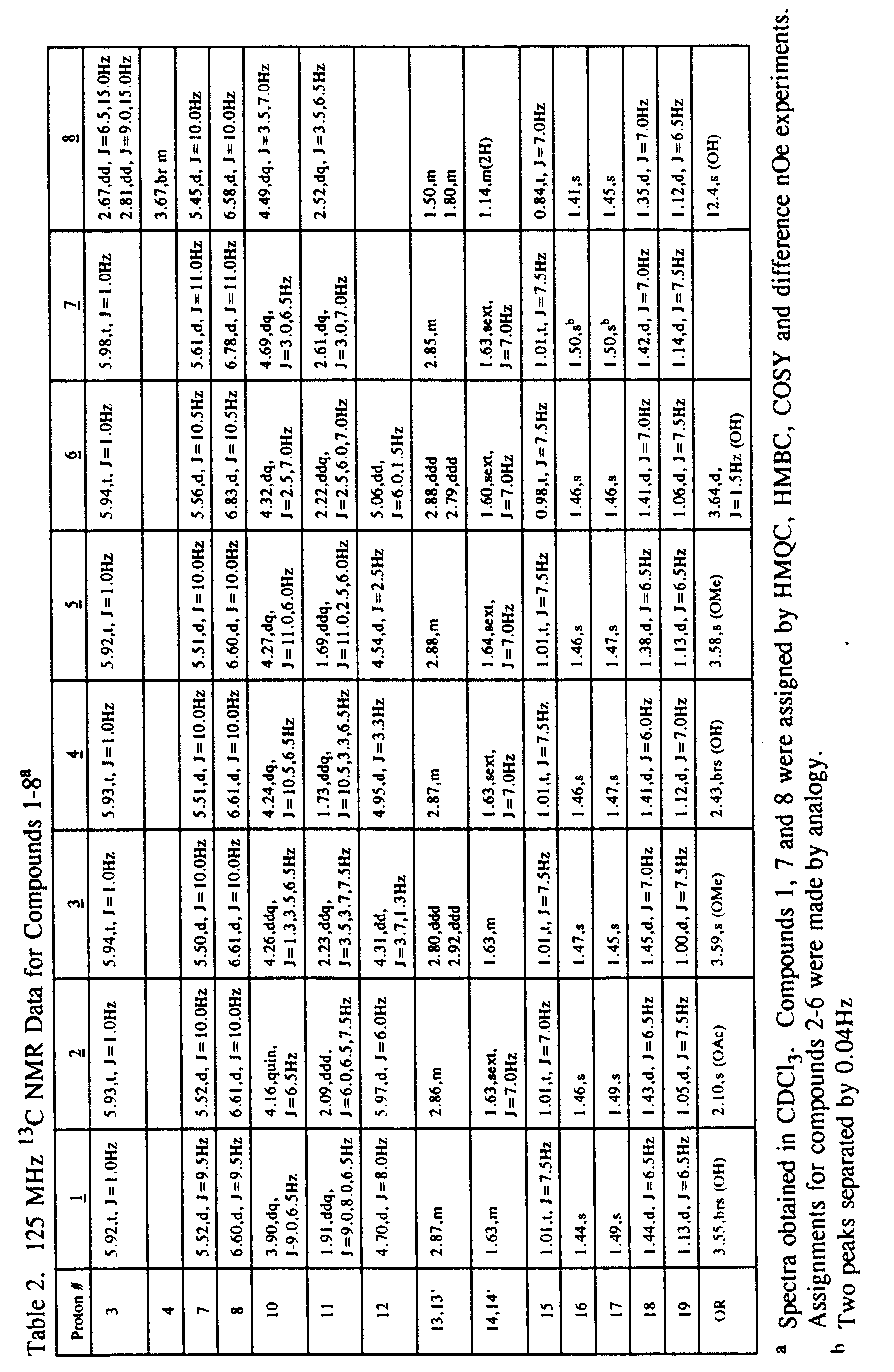

**Table 2.**

| 125 MHz ¹³C NMR Data for Compounds 1-8^{a} | | | | | | | | |
|---|---|---|---|---|---|---|---|---|
| Carbon # | 1 | 2 | 3 | 4 | 5 | 6 | 7 | 8 |
| 2 | 160.4 | 160.0 | 160.9 | 160.9 | 160.8 | 160.8 | 160.0 | 178.6 |
| 3 | 110.1 | 110.9 | 110.7 | 110.3 | 110.3 | 111.1 | 111.4 | 38.5 |
| 4 | 158.9 | 157.7 | 158.2 | 158.7 | 158.5 | 158.6 | 158.1 | 30.5 |
| 4a | 104.0 | 101.1^{b} | 102.7 | 103.5 | 103.2 | 103.5^{b} | 102.7 | 108.9 |
| 4b | 151.1 | 151.7 | 151.6 | 151.4 | 151.4 | 150.6 | 157.6 | 157.3 |
| 6 | 76.6 | 77.8 | 77.6 | 77.7 | 77.6 | 78.8 | 78.9 | 78.2 |
| 7 | 126.9 | 126.8 | 126.6 | 126.7 | 126.6 | 126.9 | 128.2 | 125.6 |
| 8 | 116.5 | 116.4 | 116.6 | 116.5 | 116.6 | 115.7 | 115.0 | 115.6 |
| 8a | 106.3 | 104.1^{b} | 104.1 | 106.1^{b} | 104.7^{b} | 102.9^{b} | 104.1 | 102.6 |
| 8b | 153.1 | 152.6 | 151.6 | 153.9 | 153.8 | 152.6 | 160.0 | 160.0^{f} |
| 10 | 77.7 | 76.6 | 73.8 | 73.0 | 73.4 | 75.6 | 77.4 | 76.1 |
| 11 | 40.5 | 38.6^{c} | 35.1 | 38.6^{c} | 38.66 | 35.1 | 45.7 | 44.2 |
| 12 | 67.2 | 67.1 | 77.6 | 61.9 | 70.8 | 65.9 | 192.9 | 201.0 |
| 12a | 106.3 | 106.2^{b} | 106.4 | 106.2^{b} | 106.0^{b} | 109.2^{b} | 106.8 | 101.2 |
| 12b | 154.5 | 154.4 | 155.1 | 153.1 | 153.1 | 154.6 | 154.3 | 160.0^{f} |
| 13 | 38.7 | 38.1^{c} | 38.6 | 38.2^{c} | 38.65 | 38.9 | 38.9 | 35.4 |
| 14 | 23.3 | 23.3 | 23.3 | 23.3 | 23.3 | 23.2 | 23.0 | 20.7 |
| 15 | 14.0 | 14.0 | 14.0 | 14.0 | 14.1 | 14.0 | 13.9 | 14.0 |
| 16 | 27.4 | 27.8 | 27.7 | 27.8 | 27.8 | 28.2 | 28.0 | 28.1 |
| 17 | 28.0 | 28.0 | 27.9 | 27.7 | 27.9 | 28.4 | 28.1 | 28.5 |
| 18 | 18.9 | 19.2 | 19.5 | 18.9 | 19.2 | 16.8 | 15.9 | 16.2 |
| 19 | 15.1 | 15.3 | 17.0 | 12.5 | 13.3 | 7.2 | 9.0 | 9.3 |
| | | 170.7^{d} | 57.6^{e} | | 59.4^{e} | | | |
| | | 21.2^{d} | | | | | | |

| | | | | | | | | |
|---|---|---|---|---|---|---|---|---|
| a Spectra recorded in CDCl₃ and attached protons determined by the DEPT pulse sequence. | | | | | | | | |
| ^{b,c} Resonances within a column may be interchangeable. | | | | | | | | |
| ^{d} Acetyl resonances. | | | | | | | | |
| ^{e} Methoxy resonances. | | | | | | | | |
| ^{f} In CD₃OD these signals appeared as doubled peaks at δ 161.20, 161.16 and δ 161.10, 161.03. | | | | | | | | |

### Example 3

This example illustrates the antiviral activity of calanolides and related compounds from Calophyllum lanigerum. Pure compounds were initially evaluated for antiviral activity using an XTT-tetrazolium anti-HIV primary screening assay described previously (Boyd, M.R., in AIDS Etiology, Diagnosis, Treatment and Prevention (DeVita V.T., Jr., Hellman S., Rosenberg S.A., eds.), pp 305-319 (Philadelphia: Lippincott, 1988); Gustafson, K.R., et al., J. Med. Chem. (in press); Weislow, O.S., et al., J. Natl. Cancer Inst., 81: 577-586 (1989); Gulakowski, R.J., et al., J. Virol. Methods, 33: 87-100 (1991)). The CEM-SS human lymphocytic target cell line used in all assays was maintained in RPMI 1640 medium (Gibco, Grand Island, NY), without phenol red, and was supplemented with 5% fetal bovine serum, 2 mM L-glutamine and 50 µg/ml gentamicin (complete medium).

Exponentially-growing cells were pelleted and resuspended at a concentration of 2.0 x 10⁵ cells/ml in complete medium. The Haitian variant of HIV, HTLV-III_{RF} (3.54 x 10⁶ SFU/ml), was used throughout. Frozen virus stock solutions were thawed immediately before use and resuspended in complete medium to yield 1.2 x 12⁵ SFU/ml. The appropriate amounts of the pure compounds for anti-HIV evaluations were dissolved in 100% dimethylsulfoxide (DMSO), then diluted in complete medium to the desired initial concentration (and with the final DMSO content not exceeding 1%). All serial drug dilutions, reagent additions, and plate-to-plate transfers were carried out with an automated Biomek 1000 Workstation (Beckman Instruments, Palo Alto, CA).

Over a broad concentration range (<0.1 - >10µM), calanolide A (**1**) provided complete protection against the cytopathic effects of HIV-1 infection in the primary screening assay and essentially halted HIV-1 reproduction in human T-lymphoblastic (CEM-SS) cells. Calanolide B (**4**), the C12 epimer of compound **1**, also provided complete inhibition of HIV-1 in this assay, giving an EC₅₀= 0.4 µM and an IC₅₀= 15.0 µM. The ester derivative, 12-acetoxycalanolide A (**2**) also was active against HIV-1, albeit somewhat less potent (EC₅₀ = 2.7 µM, IC₅₀ = 13 µM). Compound **6** showed weak but detectable anti-HIV activity, as did the 12-methoxycalanolide B (**5**); however, the antiviral activity of the corresponding 12-methoxycalanolide A (**3**) was not detectable in the primary screen. Compounds **7** and **8** were inactive in the primary screening assay, indicating the requirement for a fully reduced oxygen functionality, or derivative substitutent thereof, at C-12, and the further requirement for the intact, characteristic central carbon skeleton shared by compounds **1-6**.

For a further demonstration of the anti-HIV activity of pure compounds, a battery of interrelated assays was performed on individual wells of 96-well microtiter plates as described in detail elsewhere (Gulakowski, R.J. et al., J. Virol. Methods, 33: 87-100 (1991)). Briefly, the procedure was as follows. Uninfected CEM-SS cells were plated at a density of 1 x 10⁴ cells in 50 µl of complete medium. Diluted HIV-1 virus was then added to appropriate wells in a volume of 50 µl to yield a multiplicity of infection of 0.6. Appropriate cell, virus and drug controls were incorporated in each experiment; the final volume in each microtiter well was 200 µl. Quadruplicate wells were used for virus-infected cells, and duplicate wells were used for uninfected cells. Plates were incubated at 37°C in an atmosphere containing 5% CO₂ for 6 days. Subsequently, aliquots of cell-free supernatant were removed from each well using the Biomek, and analyzed for reverse transcriptase activity, p24 antigen production and synthesis of infectious virions as described (Gulakowski, R.J., et al., J. Virol. Methods, 33: 87-100 (1991)). Cellular growth or viability then was estimated on the remaining contents of each well using the XTT (Weislow, O.S., et al., J. Natl. Cancer Inst., 81: 577-586 (1989)), BCECF (Rink, T.L., et al., J. Cell. Biol., 95: 189-196 (1982)) and DAPI (McCaffrey, T.A., et al., In Vitro Cell Develop. Biol., 24: 247-252 (1988)) assays as described (Gulakowski, R.J., et al., J. Virol. Methods, 33: 87-100 (1991)) . To facilitate graphical displays and comparisons of data, the individual experimental assay results (of at least quadruplicate determinations of each) were averaged, and the mean values were used to calculate percentages in reference to the appropriate controls. Standard errors of the mean values used in these calculations typically averaged less than 10% of the respective mean values.

As illustrated in Figures 4A-D, calanolide A was capable of complete inhibition of the cytopathic effects of HIV-1 upon CEM-SS human lymphoblastoid target cells in vitro (EC₅₀ ⁻ 0.1 µM); direct cytotoxicity of the compound upon the target cells was apparent only at 100-fold greater concentrations (IC₅₀⁻ 13 µM; in vitro "therapeutic index" ≥ 130). Calanolide A also strikingly inhibited the production of RT, p24, and SFU in HIV-1-infected CEM-SS within these same inhibitory effective concentrations, indicating a cessation of viral replication. Similar results (data not shown) as those depicted for calanolide A in Figures 4A-D were also obtained with calanolide B, with a noncytotoxic concentration of the latter completely inhibiting HIV-replication and affording complete protection against HIV cytopathicity.

### Example 4

This example illustrates antiviral calanolide derivatives. One skilled in the art will appreciate that other antiviral calanolides or antiviral derivatives thereof, in addition to the calanolides and derivatives of Example 2, may be isolated from natural sources and/or be synthesized chemically. Antiviral calanolides or antiviral derivatives thereof can comprise two distinct series, as illustrated more generally in Figure 5. For example, calanolide A (1) from Example 2 is of series 1, wherein R¹ is CH₂CH₂CH₃, R² is 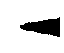 OH, R⁴ is 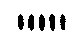 CH₃, and R⁵ is 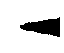 CH₃. Calanolide B (4) from Example 2 is of series 1, wherein R¹ is CH₂CH₂CH₃, R² is 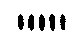 OH, R⁴ is 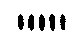 CH₃, and R⁵ is 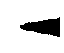 CH₃. Compound 2 from Example 2 is of series 1, wherein R¹ is CH₂CH₂CH₃, R² is 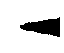 O₂CR³, R³ is -CH₃, R⁴ is 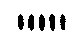 CH₃, and R⁵ is 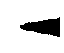 CH₃. Compound 3 from Example 2 is of series 1, wherein R¹ is CH₂CH₂CH₃, R² is 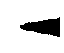 OR³, R³ is -CH₃, R⁴ is 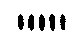 CH₃, and R⁵ is 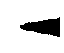 CH₃. Compound 5 from Example 2 is of series 1**,** wherein R¹ is CH₂CH₂CH₃, R² is 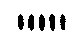 OR³, R³ is -CH₃, R⁴ is 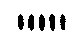 CH₃, and R⁵ is 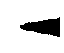 CH₃. Compound **6** from Example 2 is of series 1, wherein R¹ is CH₂CH₂CH₃, R² is 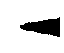 OH, and R⁴ and R⁵ are each 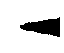 CH₃. The naturally occurring compound (Stout, G.H., J. Org. Chem., 29: 3604-3609 (1964)) costatolide (compound 9 of Figure 2) is an example of series 1, wherein R¹ is CH₂CH₂CH₃, R² is 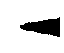 OH, R⁴ is 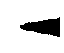 CH₃, and R⁵ is 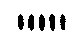 CH₃. Compound 7 from Example 2 illustrates a related natural product from Calophyllum lanigerum which has the same carbon skeleton that characterizes members of series 1, wherein R⁴ and R⁵ are each 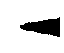 CH₃.

Whereas the above examples provide the essential precedents for the characteristic structural (stereochemical and substituent) features about C-10, C-11, and C-12 relative to the antiviral calanolides and antiviral derivatives thereof of Figure 5, there are also precedents for variations at R¹ provided by related naturally occurring compounds having variations at C-4. For example, compounds, which have the same central carbon skeleton that characterize the antiviral calanolides and antiviral derivatives thereof of Figure 5, have been isolated that have CH₃ or aryl substituents at C-4 (Dharmaratne, H.R.W., et al., Phytochemistry, 24: 1553-1556 (1985); Gunasekera, S.P., et al., J. Chem. Soc., 1505-1511 (1977)), but differ in the stereochemistry and/or substituents at C-10, C-11, and C-12 that characterize the calanolides.

Whereas the above examples provide precedents for the isolation of naturally occurring antiviral calanolides and antiviral derivatives thereof, and of other natural products that show the essential structural features of antiviral calanolides and derivatives thereof of series 1, one skilled in the art also will appreciate that, using standard organic chemical methodology, a number of structural modifications can be made for purposes of preparing antiviral calanolides or antiviral derivatives thereof. For example, antiviral calanolides and antiviral derivatives of series 2 can be made from calanolides or derivatives thereof of series 1. More specifically, an antiviral member of series 1 may be converted to the corresponding member of series 2, wherein C7 and C8 of the latter are fully saturated. 7,8-dihydro calanolide A of series 2 can be made from calanolide A of series 1 by platinum oxide-catalyzed hydrogenation of the 7,8 olefinic linkage.

As a further example, an antiviral member of either series 1 or 2, wherein R² is 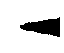 OH or 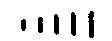 OH can be converted to the corresponding C-12 ester or sulfonate ester, wherein R² is 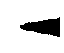 O₂CR³, 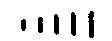 O₂CR³, 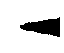 O₃SR³, or 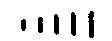 O₃SR³, by reaction with the corresponding acid halide, X-OCR³, or X-O₂SR³, wherein X = Cl, Br, or I, and R³ is C₁-C₆ alkyl or aryl, or, alternatively, by reaction directly with the corresponding acids, HO₂CR³, or HO₃SR³, wherein R³ is C₁-C₆ alkyl or aryl, and dicyclohexylcarbodiimide in anhydrous pyridine or triethylamine. Vice versa, a C-12 ester of either series 1 or 2, wherein R² is 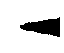 O₂CR³, 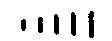 O₂CR³, 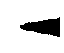 O₃SR³ or 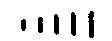 O₃SR³, and R³ is C₁-C₆ alkyl or aryl, may be hydrolyzed chemically or enzymatically to the respective parent calanolide compound, wherein R² is 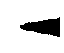 OH or 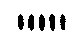 OH. It is further noted that calanolide derivatives esterified at C-12, which are susceptible to plasma- or tissue-esterase mediated deesterification, can serve as prodrugs in vivo for antiviral calanolides, wherein the C-12 substituent is 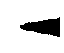 OH or 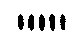 OH.

All publications referenced herein are hereby incorporated by reference in their entireties.

While this invention has been described with emphasis upon preferred embodiments, it will be obvious to those of ordinary skill in the art that the preferred compounds, compositions, and methods may be varied. It is intended that the invention may be practiced otherwise than as specifically described herein. Accordingly, this invention includes all modifications encompassed within the spirit and scope of the following claims.

## Claims

1. A compound, in substantially pure form, selected from the group consisting of:

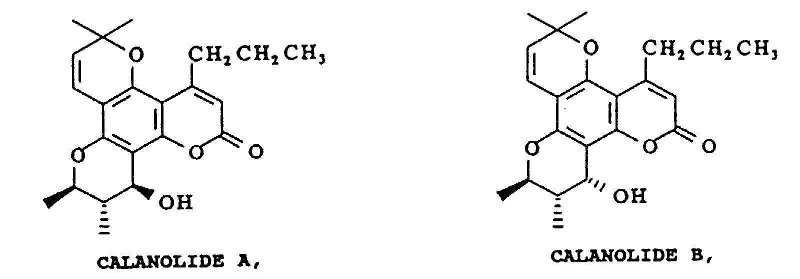

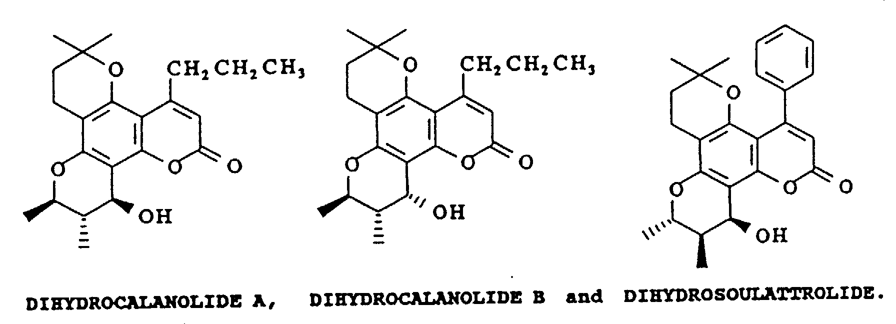

2. The compound of claim 1, in substantially pure form, which is:

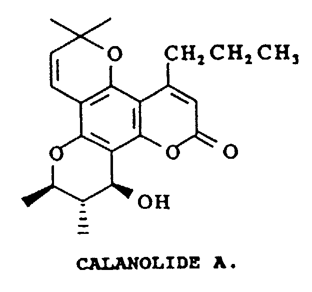

3. The compound of claim 1, in substantially pure form, which is:

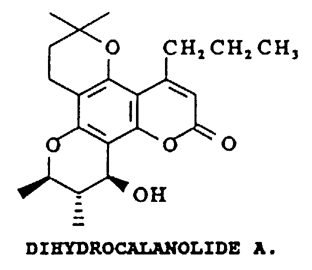

4. The compound of claim 1, in substantially pure form, which is:

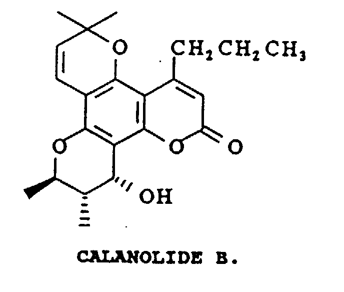

5. The compound of claim 1, in substantially pure form, which is:

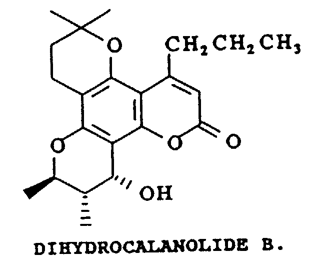

6. The compound of claim 1, in substantially pure form, which is:

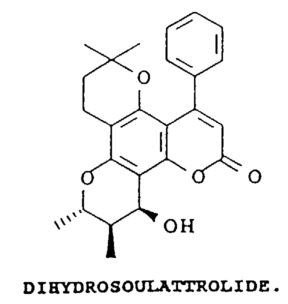

7. A method of isolating calanolides from plants of the genus Calophyllum, which method comprises the steps of:
(a) extracting dried plant material with an organic solvent to obtain a crude extract;
(b) solvent-solvent partitioning said crude extract to concentrate calanolides in a nonpolar fraction;
(c) subjecting said nonpolar fraction to gel permeation or vacuum liquid chromatography; and
(d) isolating and purifying said calanolides by HPLC on silica gel and C₁₈ reversed-phase columns.

8. The method of Claim 7, wherein said plant material is Calophyllum lanigerum.

9. An antiretroviral composition which comprises a pharmaceutically acceptable carrier and an antiretrovirally effective amount of at least one compound selected from the group consisting of: 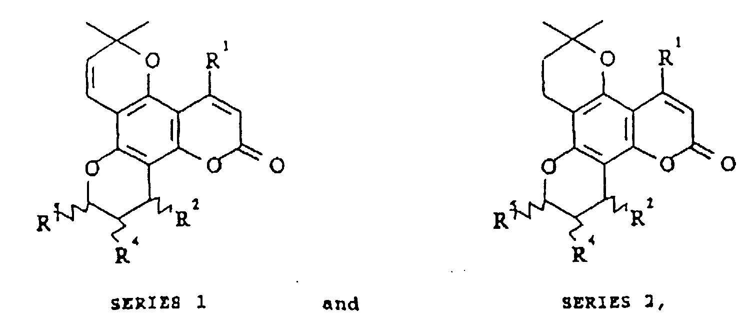 wherein R¹ is C₁-C₆ alkyl or aryl; R² is 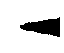 OH, 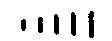 OH, 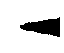 OR³, 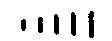 OR³, 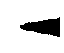 O₂CR³, 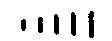 O₂CR³, 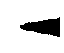 O₃SR³, or 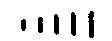 O₃SR³, wherein R³ is C₁-C₆ alkyl or aryl; and R⁴ and R⁵ are the same or different and are each 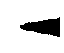 CH₃ or 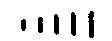 CH₃.

10. The composition of claim 9, wherein said compound is selected from the group consisting of:

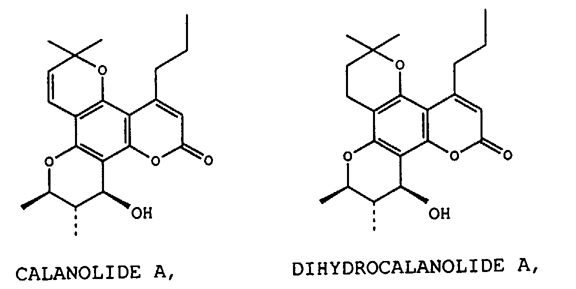

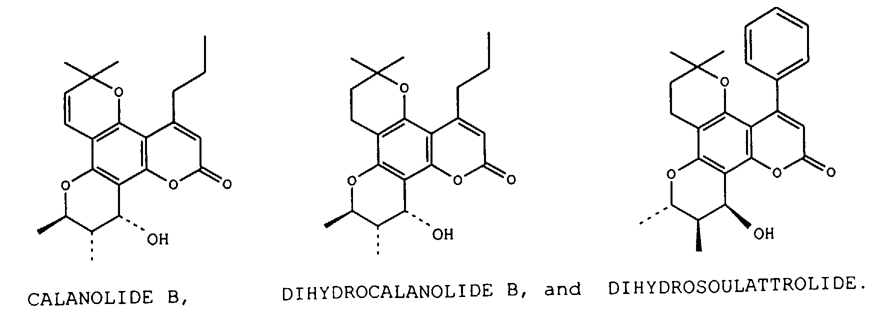

11. The composition of claim 10, wherein said compound is selected from the group consisting of calanolide A, dihydrocalanolide A, calanolide B, and dihydrocalanolide B.

12. The composition of any one of claims 9 to 11, which further comprises an antivirally effective amount of at least one additional antiviral compound other than a compound selected from the group consisting of:

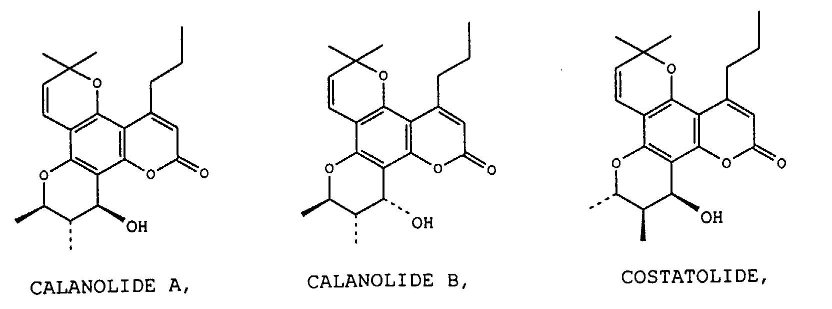

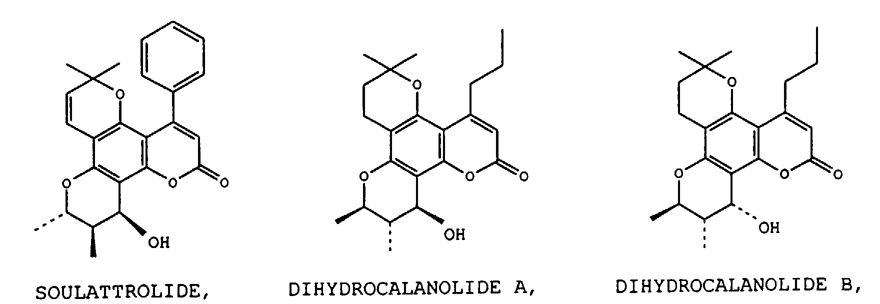

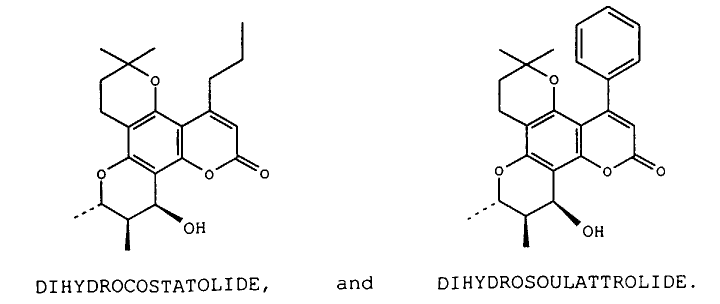

13. A compound that is capable of inhibiting a retrovirus that contains a reverse transcriptase which is inhibited by the compound, for use in a method of preventing or treating a retroviral infection, said compound being selected from the group consisting of: 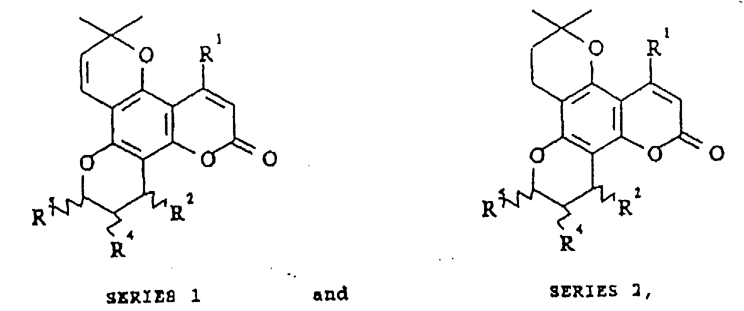 wherein R¹ is C₁-C₆ alkyl or aryl; R² is 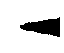 OH, 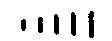 OH, 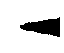 OR³, 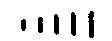 OR³, 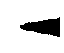 O₂CR³, 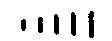 O₂CR³, 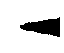 O₃SR³, or 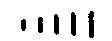 O₃SR³, wherein R³ is C₁-C₆ alkyl or aryl; and R⁴ and R⁵ are the same or different and are each 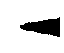 CH₃ or 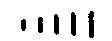 CH_{3.}

14. The compound of claim 13 selected from the group consisting of:

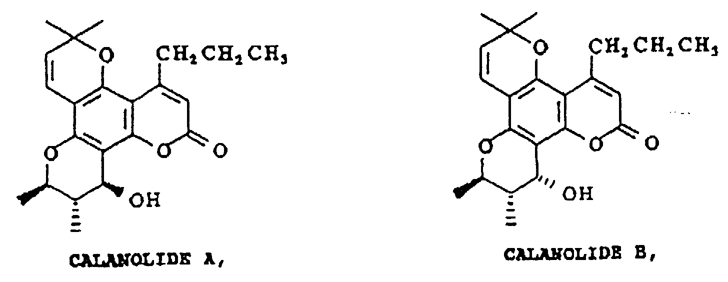

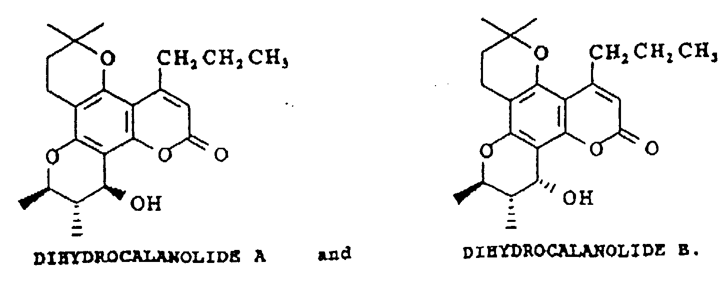

15. Use of compound that is capable of inhibiting a retrovirus that contains a reverse transcriptase which is inhibited by the compound, in the manufacture of a medicament for preventing or treating a retroviral infection, said compound being selected from the group consisting of: 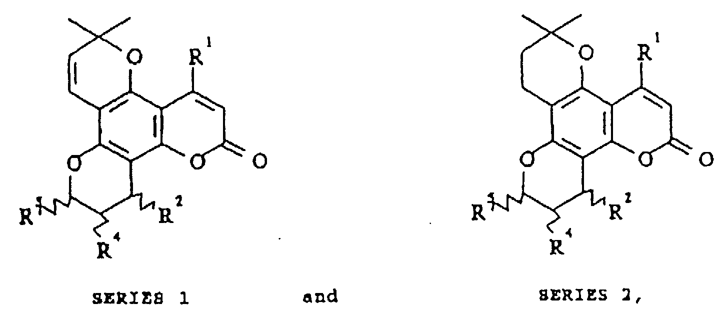 wherein R¹ is C₁-C₆ alkyl or aryl; R² is 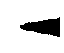 OH, 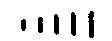 OH, 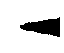 OR³, 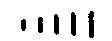 OR³, 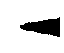 O₂CR³, 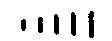 O₂CR³, 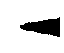 O₃SR³, or 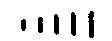 O₃SR³, wherein R³ is C₁-C₆ alkyl or aryl; and R⁴ and R⁵ are the same or different and are each 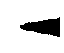 CH₃ or 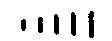 CH₃.

16. Use according to claim 15 wherein the medicament is for co-administration with an antivirally effective amount of at least one additional antiviral compound other than a compound selected from the group consisting of Series 1 and Series 2 of Claim 15.

17. Use according to claim 15 or claim 16, wherein said retroviral infection is by a retrovirus that contains a reverse transcriptase which is inhibited by the antiretroviral compound.

18. Use according to any one of claims 15 to 17, wherein said retrovirus is a human immunodeficiency virus.

19. Use according to any one of claims 15 to 18, wherein said compound is selected from the group consisting of:

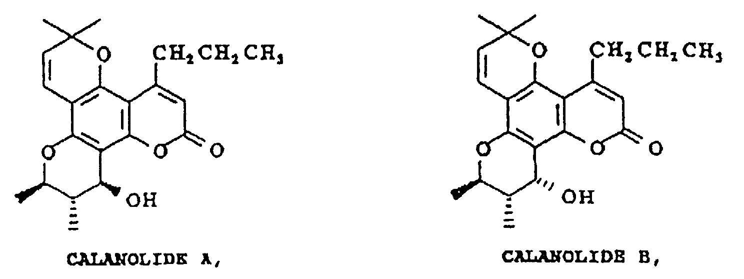

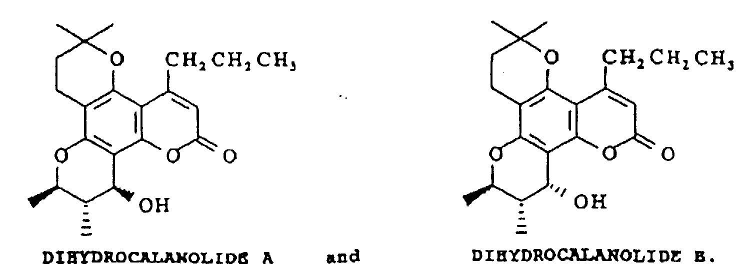

## Patentansprüche

1. Verbindung in im wesentlichen reiner Form, ausgewählt aus der aus: 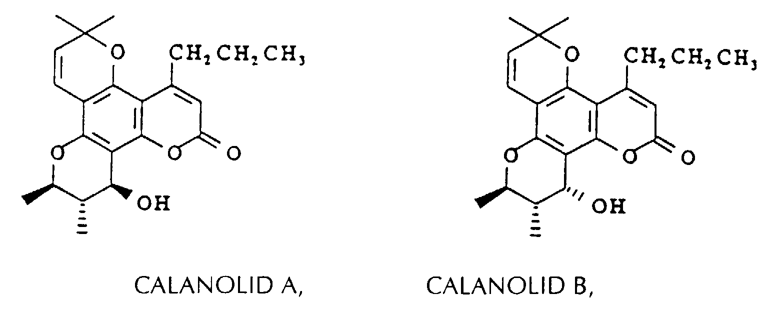 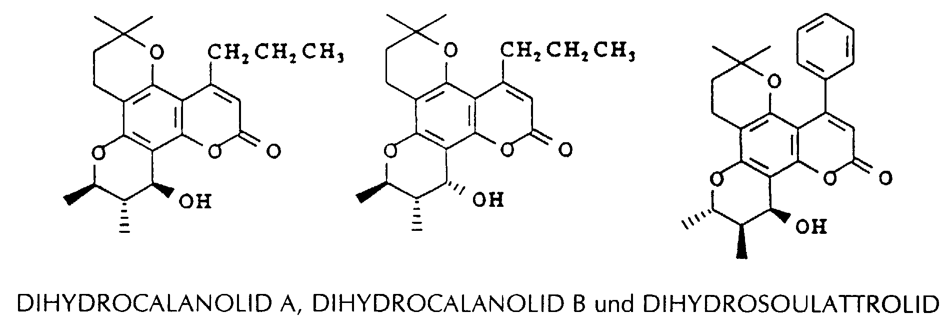 bestehenden Gruppe.

2. Verbindung nach Anspruch 1 in im wesentlichen reiner Form, die 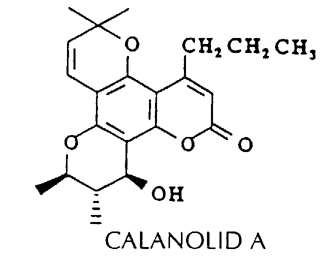 ist.

3. Verbindung nach Anspruch 1 in im wesentlichen reiner Form, die 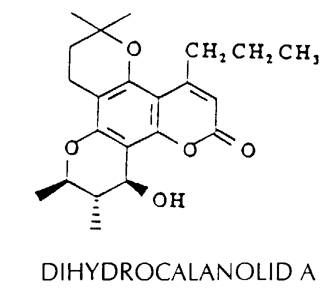 ist.

4. Verbindung nach Anspruch 1 in im wesentlichen reiner Form, die 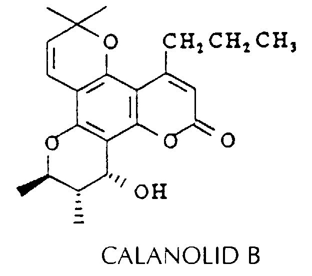 ist.

5. Verbindung nach Anspruch 1 in im wesentlichen reiner Form, die 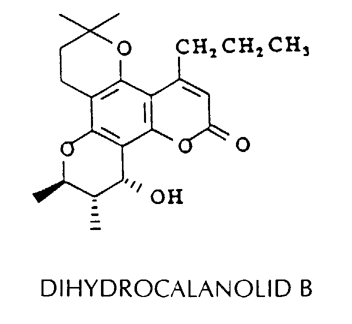 ist.

6. Verbindung nach Anspruch 1 in im wesentlichen reiner Form, die 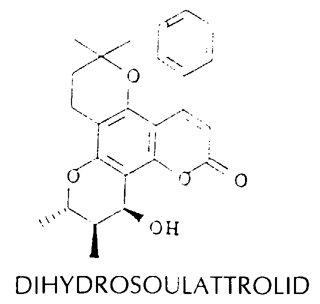 ist.

7. Verfahren zum Isolieren von Calanoliden aus Pflanzen der Art Calophyllum, wobei das Verfahren folgende Schritte umfaßt:
(a) Extrahieren von getrocknetem Pflanzenmaterial mit einem organischen Lösungsmittel, um einen Rohextrakt zu erhalten;
(b) Lösungsmittel-Lösungmittel-Trennung des Rohextraktes, um Calanolide in einer nichtpolaren Fraktion aufzukonzentrieren;
(c) Unterziehen der nichtpolaren Fraktion der Gelpermeation oder Vakuum-Flüssigchromatographie; und
(d) Isolieren und Reinigen der Calanolide mittels HPLC auf Silikagel und C₁₈-Umkehrphasensäulen.

8. Verfahren nach Anspruch 7, worin das Pflanzenmaterial Calophyllum lanigerum ist.

9. Antiretrovirale Zusammensetzung, die eine antiretroviral wirksame Menge zumindest einer Verbindung, ausgewählt aus der Gruppe, bestehend aus: 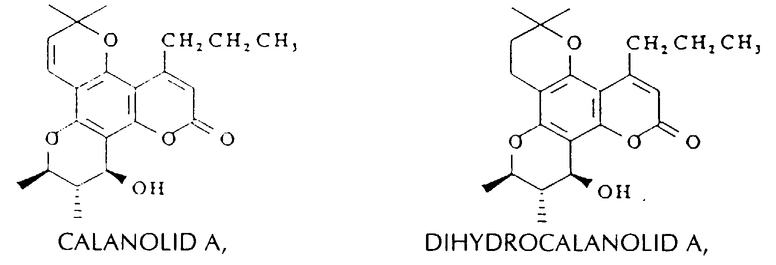 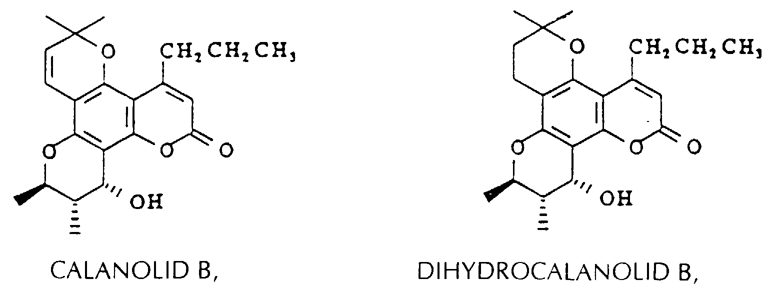 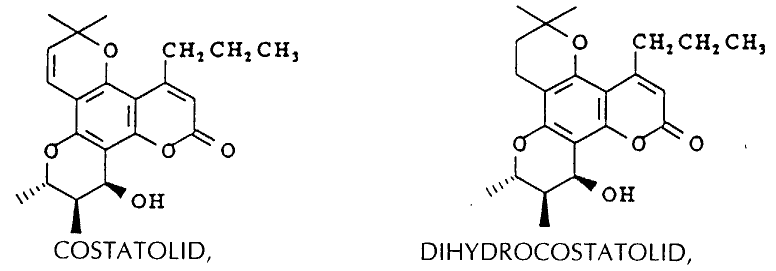 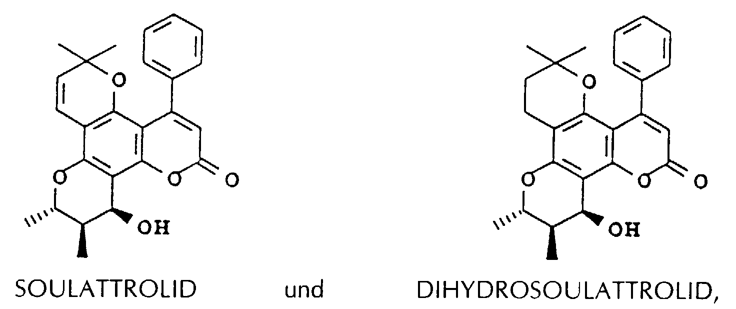 sowie einen pharmazeutisch annehmbaren Träger umfaßt.

10. Zusammensetzung nach Anspruch 9, worin die Verbindung aus der aus Calanolid A, Dihydrocalanolid A, Calanolid B, Dihydrocalanolid B und Dihydrosoulattrolid bestehenden Gruppe ausgewählt ist.

11. Zusammensetzung nach Anspruch 10, worin die Verbindung aus der aus Calanolid A, Dihydrocalanolid A, Calanolid B und Dihydrocalanolid B bestehenden Gruppe ausgewählt ist.

12. Zusammensetzung nach Anspruch 10, die weiters eine antiviral wirksame Menge zumindest einer zusätzlichen antiviralen Verbindung mit Ausnahme einer Verbindung, ausgewählt aus der aus Calanolid A, Calanolid B, Costatolid, Soulattrolid, Dihydrocalanolid A, Dihydrocalanolid B, Dihydrocostatolid und Dihydrosoulattrolid bestehenden Gruppe, umfaßt.

13. Verbindung, die zur Hemmung eines Retrovirus fähig ist, das eine Reverse Transkriptase enthält, die durch die Verbindung gehemmt wird, zur Verwendung in einem Verfahren zur Prävention oder Behandlung einer retroviralen Infektion, wobei die Verbindung aus der aus: 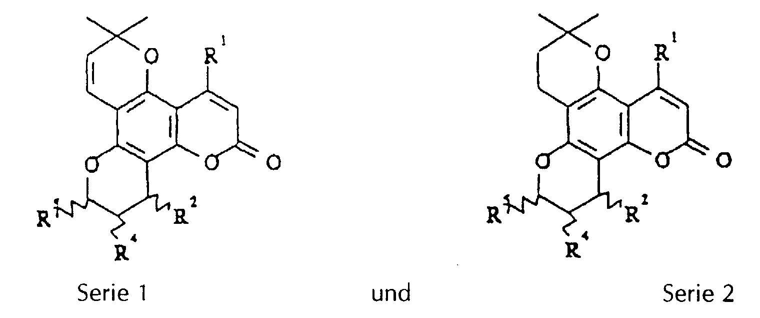 bestehenden Gruppe ausgewählt ist, worin R¹ = C₁-C₆-Alkyl oder Aryl ist; R²= 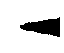 OH, 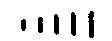 OH, 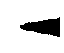 OR³, 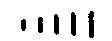 OR³, 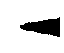 O₂CR³, 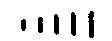 O₂CR³, 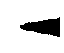 O₃SR³, oder 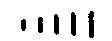 O₃SR³, ist, worin R³ = C₁-C₆-Alkyl oder Aryl ist; und R⁴ und R⁵ gleich oder voneinander verschieden sind und jeweils 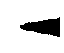 CH₃ oder 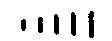 CH₃ sind.

14. Verbindung nach Anspruch 13, ausgewählt aus der aus: 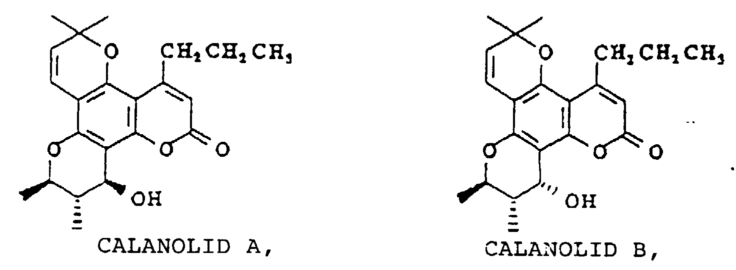 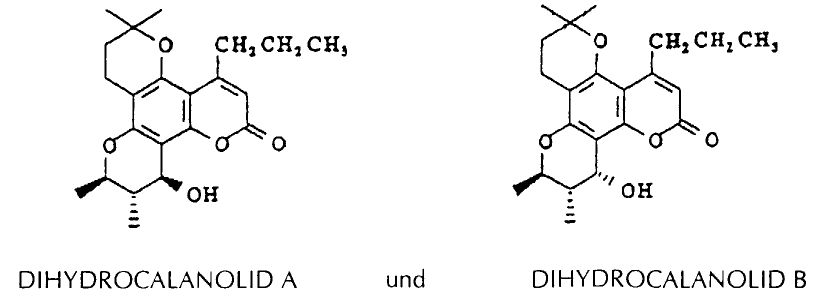 bestehenden Gruppe.

15. Verwendung einer Verbindung, die zur Hemmung eines Retrovirus fähig ist, das eine Reverse Transkriptase enthält, die durch die Verbindung gehemmt wird, zur Herstellung eines Medikaments zur Prävention oder Behandlung einer retroviralen Infektion, wobei die Verbindung aus der aus: 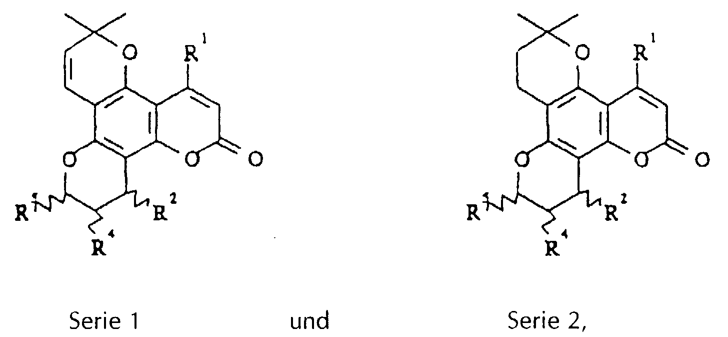 bestehenden Gruppe ausgewählt ist, worin R¹ = C₁-C₆-Alkyl oder Aryl ist; R²= 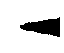 OH, 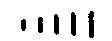 OH, 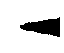 OR³, 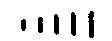 OR³, 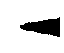 O₂CR³, 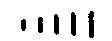 O₂CR³, 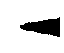 O₃SR³, oder 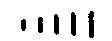 O₃SR³, ist, worin R³ = C₁-C₆-Alkyl oder Aryl ist; und R⁴ und R⁵ gleich oder voneinander verschieden sind und jeweils 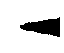 CH₃ oder 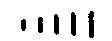 CH₃ sind.

16. Verwendung nach Anspruch 15, worin das Medikament zur gleichzeitigen Verabreichung mit einer antiviral wirksamen Menge zumindest einer zusätzlichen antiviralen Verbindung mit Ausnahme einer Verbindung, ausgewählt aus der aus Serie 1 und Serie 2 nach Anspruch 15 bestehenden Gruppe, bestimmt ist.

17. Verwendung nach Anspruch 15 oder 16, worin die retrovirale Infektion durch ein Retrovirus erfolgt, das eine Reverse Transkriptase enthält, die durch die antiretrovirale Verbindung gehemmt wird.

18. Verwendung nach einem der Ansprüche 15 bis 17, worin das Retrovirus ein menschliches Immundefizienz-(HI-)Virus ist.

19. Verwendung nach einem der Ansprüche 15 bis 18, worin die Verbindung aus der aus: 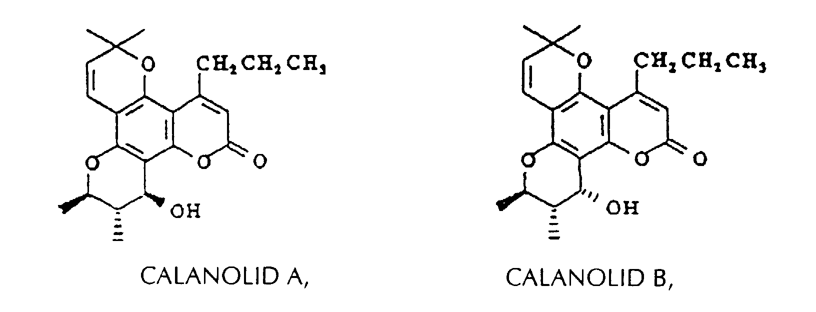 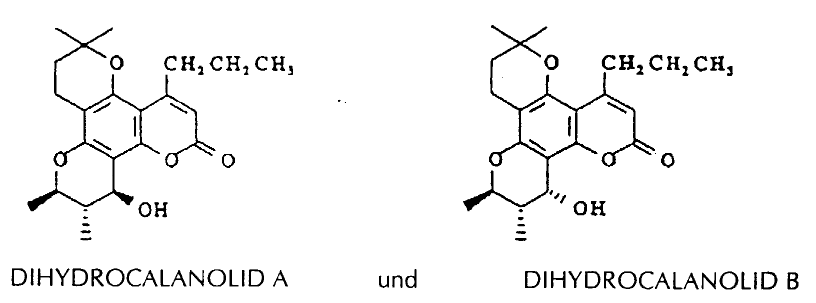 bestehenden Gruppe ausgewählt ist.

## Revendications

1. Composé, sous une forme sensiblement pure, sélectionné dans le groupe consistant en :

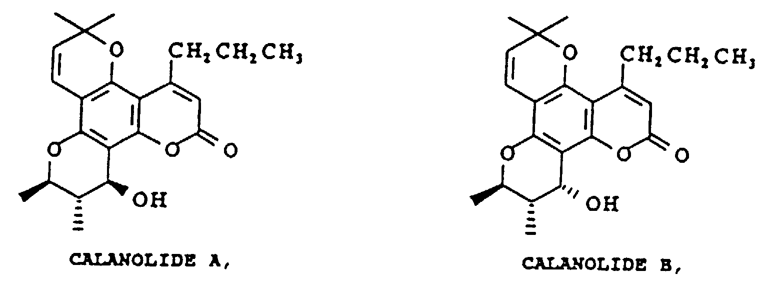

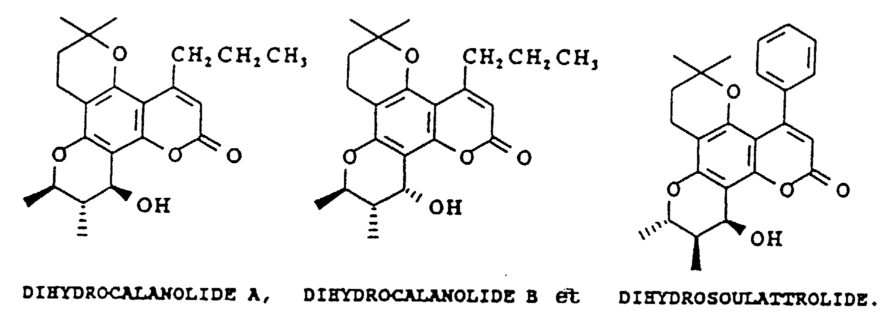

2. Composé de la revendication 1, sous une forme sensiblement pure, qui est :

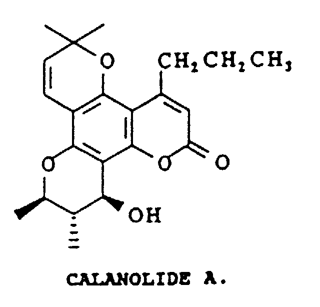

3. Composé de la revendication 1, sous une forme sensiblement pure, qui est :

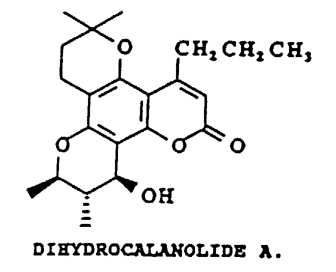

4. Composé de la revendication 1, sous une forme sensiblement pure, qui est :

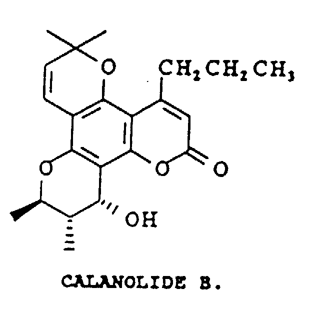

5. Composé de la revendication 1, sous une forme sensiblement pure, qui est :

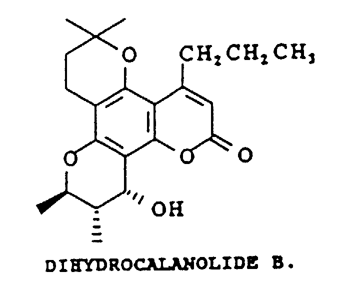

6. Composé de la revendication 1, sous une forme sensiblement pure, qui est :

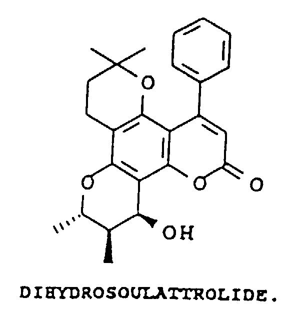

7. Méthode d'isolement de calanolides de plantes du genre Calophyllum, laquelle méthode comprend les étapes de :
(a) extraire le matériel de plante séchée avec un solvant organique pour obtenir un extrait brut;
(b) répartir solvant-solvant ledit extrait brut pour concentrer les calanolides en une fraction non polaire;
(c) soumettre ladite fraction non polaire à une chromatographie par perméation de gel ou liquide sous vide; et
(d) isoler et purifier lesdits calanolides par HPLC sur colonnes de gel de silice et C₁₈ en phase inverse.

8. Méthode de la revendication 7, où ledit matériel de plante est Calophyllum lanigerum.

9. Composition antirétrovirale qui comprend un support pharmaceutiquement acceptable et une quantité antirétroviralement efficace d'au moins un composé sélectionné dans le groupe consistant en : 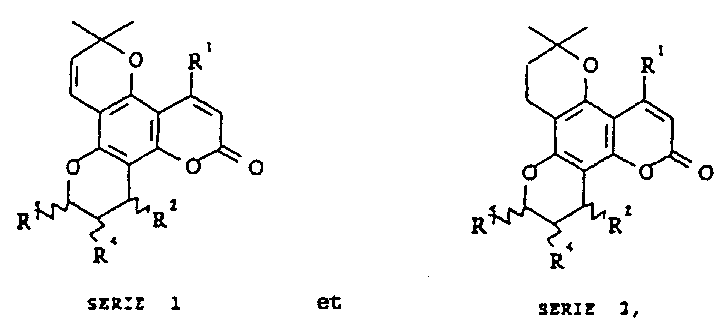 où R¹ est alkyle C₁-C₆ ou aryle; R² est 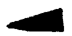 OH, 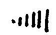 OH, 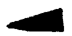 OR³, 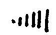 OR³, 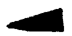 O₂CR³, 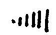 O₂CR³, 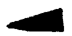 O₃SR³, ou 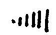 O₃SR³, où R³ est alkyle C₁-C₆ ou aryle; et R⁴ et R⁵ sont identiques ou différents et chacun est 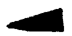 CH₃ ou 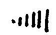 CH₃.

10. Composition de la revendication 9, où ledit composé est sélectionné dans le groupe consistant en :

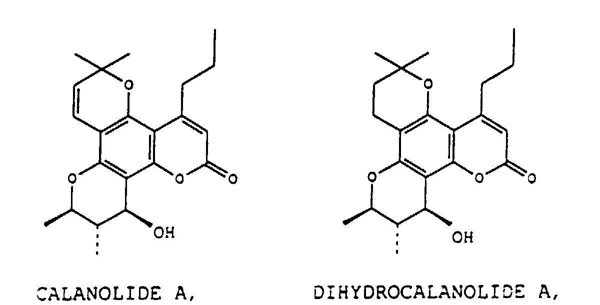

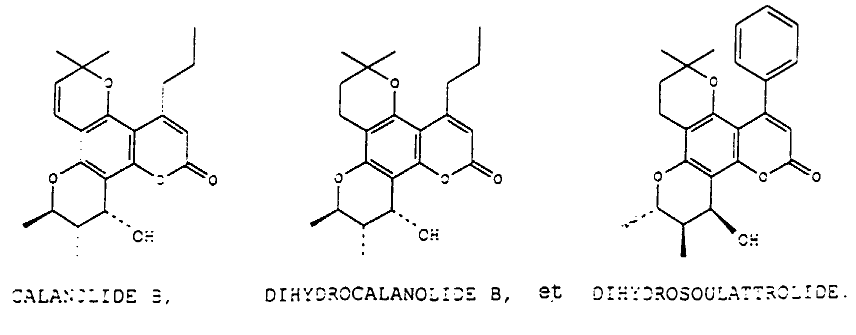

11. Composition de la revendication 10, où ledit composé est sélectionné dans le groupe consistant en calanolide A, dihydrocalanolide A, calanolide B, et dihydrocalanolide B.

12. Composition selon l'une quelconque des revendications 9 à 11, qui comprend de plus une quantité antiviralement efficace d'au moins un composé antiviral additionnel autre qu'un composé sélectionné dans le groupe consistant en :

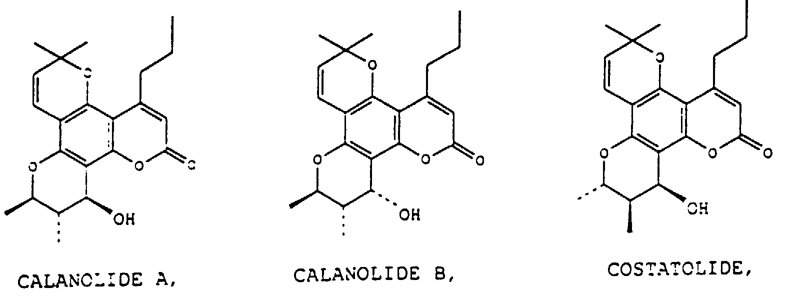

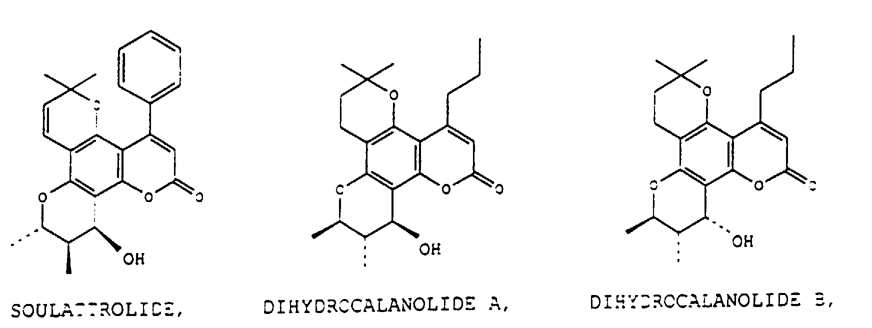

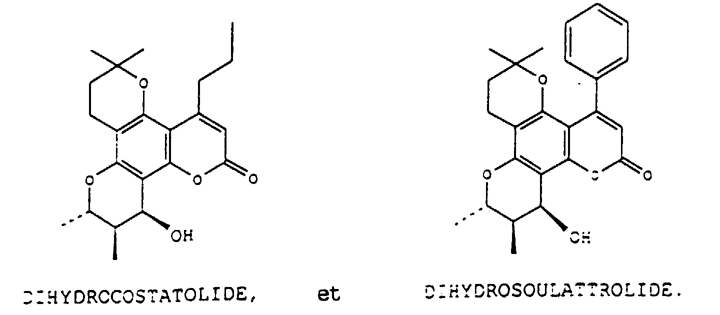

13. Composé qui est capable d'inhiber un rétrovirus qui contient une transcriptase réverse qui est inhibée par le composé, pour une utilisation dans une méthode de prévention ou de traitement d'une infection rétrovirale, ledit composé étant sélectionné dans le groupe consistant en : 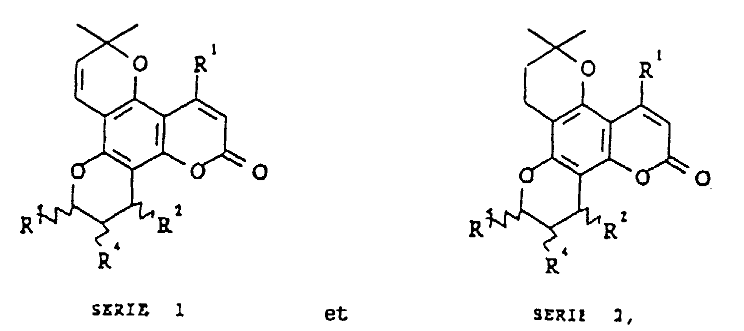 où R¹ est alkyle C₁-C₆ ou aryle; R² est 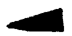 OH, 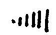 OH, 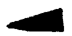 OR³, 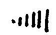 OR³, 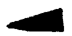 O₂CR³, 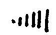 O₂CR³, 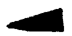 O₃SR³, ou 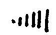 O₃SR³, où R³ est alkyle C₁-C₆ ou aryle; et R⁴ et R⁵ sont identiques ou différents et chacun est 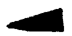 CH₃ ou 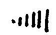 CH₃.

14. Composé de la revendication 13 sélectionné dans le groupe consistant en :

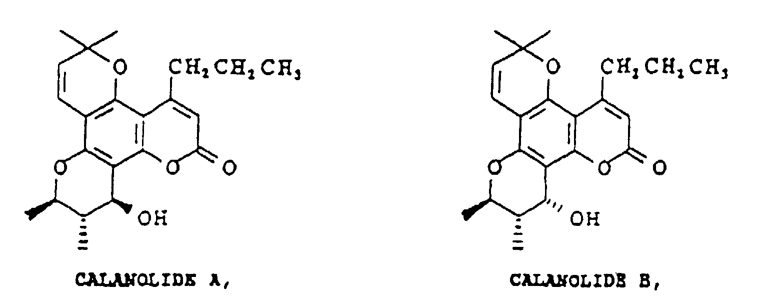

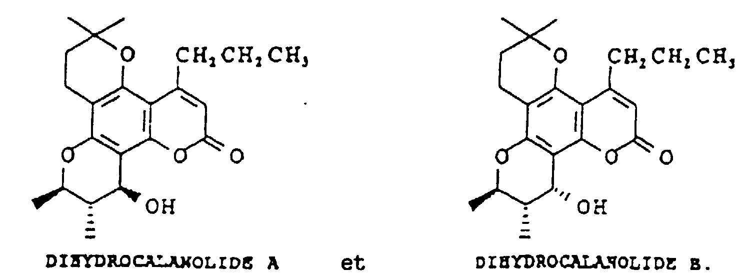

15. Utilisation d'un composé qui est capable d'inhiber un rétrovirus qui contient une transcriptase réverse qui est inhibée par le composé, dans la fabrication d'un médicament pour la prévention ou le traitement d'une infection rétrovirale, ledit composé étant sélectionné dans le groupe consistant en : 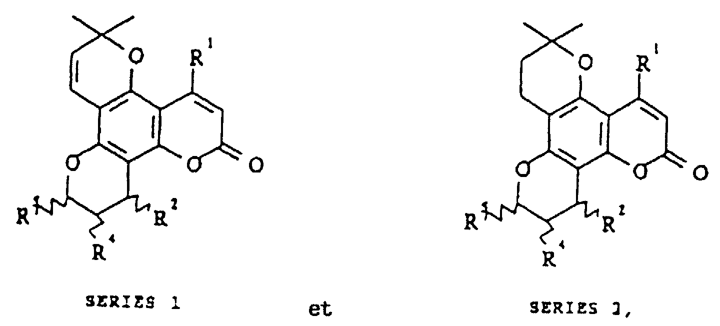 où R¹ est alkyle C₁-C₆ ou aryle; R² est 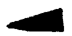 OH, 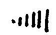 OH, 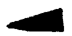 OR³, 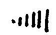 OR³, 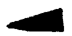 O₂CR³, 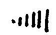 O₂CR³, 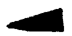 O₃SR³, ou 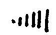 O₃SR³, où R³ est alkyle C₁-C₆ ou aryle; et R⁴ et R⁵ sont identiques ou différents et chacun est 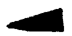 CH₃ ou 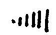 CH₃.

16. Utilisation selon la revendication 15 où le médicament est pour une co-administration avec une quantité antiviralement efficace d'au moins un composé antiviral additionnel autre qu'un composé sélectionné dans le groupe consistant en Série 1 et Série 2 de la revendication 15.

17. Utilisation selon la revendication 15 ou la revendication 16, où ladite infection rétroviralle est par un rétrovirus qui contient une transcriptase réverse qui est inhibée par le composé antirétroviral.

18. Utilisation selon l'une quelconque des revendications 15 à 17, où ledit rétrovirus est un virus d'immunodéficience humaine.

19. Utilisation selon l'une quelconque des revendications 15 à 18, où ledit composé est sélectionné dans le groupe consistant en :
